# EUROPEAN PATENT APPLICATION

(11) **EP 2 239 320 A1**
(43) Date of publication of application: **13.10.2010**
(21) Application number: 09705123.9
(22) Date of filing: 29.01.2009
(51) Int. Cl.: C12N 5/06, A61K 35/74, A61P 25/00, A61P 43/00, C07D 487/22, C07D 491/22, C07D 519/00, C07J 13/00, C07J 71/00, C07J 73/00, C12P 17/18, C12P 33/00

(54) **NERVE TRUNK CELL PROPAGATION ACCELERATOR**

(30) Priority: 29.01.2008 JP 2008017031
(71) Applicant: Kyowa Hakko Kirin Co., Ltd., Tokyo 100-8185 (JP)
(72) Inventor: ONODERA, Hideyuki c/o Med. Chem. Res. Lab., Shizuoka 411-8731 (JP); ICHIMURA, Michio c/o Kyowa, Medex Research Lab, Shizuoka 411-0932 (JP); BABA, Kouji, c/o Tokyo Branch, Tokyo 150-0013 (JP); AGATSUMA, Tsutomu c/o Med. Chem. Res. Lab., Shizuoka 411-8731 (JP); SASHO, Setsuya c/o Head Office, Kyowa Hakko Kirin Co Ltd., Tokyo 100-8185 (JP); Susuki, Makoto c/o Kyowa Wellness, Co Ltd, Tokyo 103-8503 (JP); IWAMOTO, Susumu c/o Med. Chem. Res. Lab.,, 1188, Shimotogari, Nagaisumi-cho, Sunto-gun, Shizuoka 411-8731 (JP); KAKITA, Shingo c/o Pharmacokinetic Res. Lab., 1188, Shimotogari, Nagaizumi-cho, Sunto-gun Shizuoka 411-8731 (JP)
(74) Representative: Harding, Charles Thomas
(86) International application number: PCT/JP2009/051417
(87) International publication number: WO 2009/096445

(57) **Abstract**

A proliferation promoting agent for neural stem cells, which comprises a compound produced by *Penicillium* sp. CND1007 (FERM BP-10917) or a pharmaceutically acceptable salt thereof, as an active ingredient; a novel compound produced by *Penicillium* sp. CND1007 or a pharmaceutically acceptable salt thereof; a method for producing neural stem cells, which comprises culturing a neural stem cells to proliferate the neural stem cells in a presence of the compound produced by *Penicillium* sp. CND1007 or a pharmaceutically acceptable salt thereof, and harvesting the neural stem cells from the culture; and the like are provided.

## Description

### TECHNICAL FIELD

The present invention relates to a proliferation promoting agent for stem cells and progenitor cells, which comprises a compound produced by *Penicillium* sp. CND1007 (FERM BP-10917), a related compound thereof, or a pharmaceutically acceptable salt thereof, as an active ingredient. It further relates to a compound produced by *Penicillium* sp. CND1007 or a pharmaceutically acceptable salt thereof.

### BACKGROUND ART

Neurodegenerative diseases are diseases in which cerebral and peripheral nerve cells are damaged by a hereditary factor, an environmental factor, an aging factor and the like. Specifically, they include Parkinson's disease, Alzheimer's disease, triplet repeat disease, amyotrophic lateral sclerosis, polyneuropathy, spinal cord injury, cerebrovascular accidents and the like.
Although a general therapeutic method for these neurodegenerative diseases is a method in which neurotransmitters lost by the injury of nerve cells is supplemented, the diseases for which the therapeutic method is effective is limited to Parkinson's disease, Alzheimer's disease and the like at present. Additionally, the progress of nerve cell death cannot be stopped by the neurotransmitter supplementation method.

Regenerative medicine which regenerates the central nervous system has been investigated from the viewpoint of transplantation, as a therapeutic method for positively recovering the function of dopaminergic neurons which had lost by Parkinson's disease. However, the regenerative medicine has not been generally used due to various problems caused by the use of aborted fetal brain. Additionally, studies have also been conducted on a therapeutic method in which neural stem cells obtained from a fetal brain or ES cells obtained from a human fertilized eggs are mass-cultured *in vitro* and differentiated into a neuron of interest to use it for transplantation (Stem Cells, 2006, vol. 24, p. 1583-1593; The Journal of Neuroscience, 2005, vol. 25, p. 4694-4705). However, its clinical applications are not in progress since the techniques for accurately differentiating them into the neuron of interest have not been established yet; teratomas are formed by undifferentiated cells; and there are problems caused by the use of fetal neural stem cells or human ES cells. Accordingly, a technique in which adult-derived neural stem cells are cultured *in vitro* and used for transplantation is regarded as a promising technique and search for factors which efficiently accelerate proliferation of neural stem cells is expected (Nature Reviews Neuroscience, 2006, vol. 7, p. 395 - 406).

As a low molecular compound which promotes proliferation of neural stem cells, for example, Salvianolic acid B (Japanese Published Unexamined Patent Application No. 76948/06), hedgehog signal agonists (Journal of Biology, 2002, vol. 1, p. 10), selective serotonin reuptake inhibitors (Science, 2003, vol. 301, p. 805-809; Proceedings of the National Academy of Science of the United States of America, 2006, vol. 103, p.8233-8238), metabotropic glutamate receptor antagonists (Biochemical and Biophysical Research Communications, 2004, vol. 315, p. 493-496), PPAR γ agonists (The Journal of Biological Chemistry, 2006, vol. 281, p. 12673-12681), NMDA agonists (Journal of Cell Science, 2007, vol. 120, p. 1358-1370) and the like have been reported.

On the other hand, for example, the following compounds (A), (A1) and (1) to (6) are known as sterol derivatives. As indole and oxyindole derivatives, for example, the following compounds (7) and (8) are known. As quinazoline derivatives, for example, the following compounds (9) to (11) are known (cf. Patent Literatures 1 and 2 and Non-Patent Literatures 1 to 9).

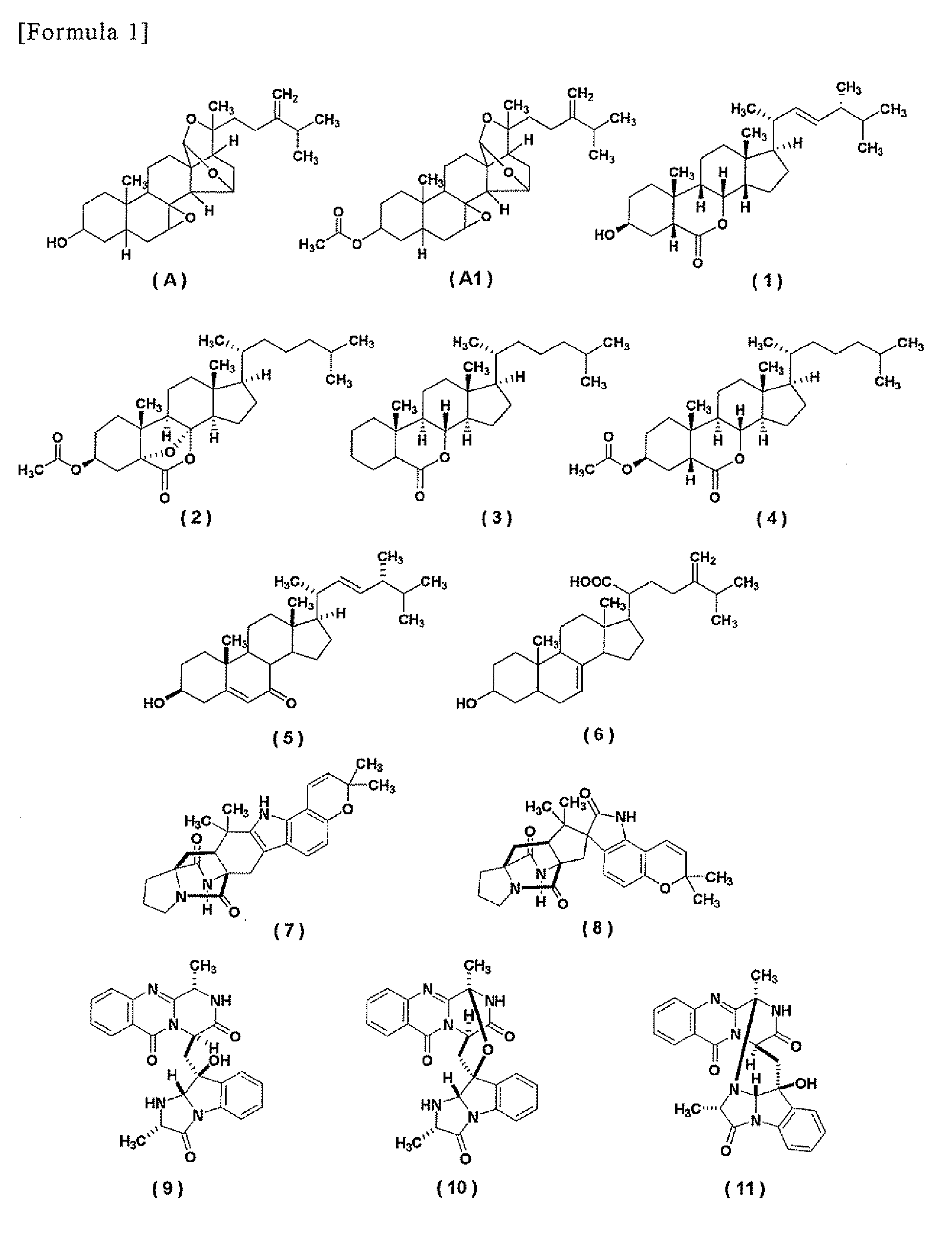

Patent Literature 1: Japanese Published Examined Patent Application No. 79080/93
Patent Literature 2: Japanese Published Examined Patent Application No. 5909/96
Non-Patent Literature 1: Heterocycles, 1985, vol. 23, p. 1607-1610
Non-Patent Literature 2: Tetrahedron Letters, 1979, vol. 33, p. 3119-3122
Non-Patent Literature 3: CAS REGISTRY Database, Registry Number: 6048-74-4
Non-Patent Literature 4: Agricultural and Biological Chemistry, 1964, vol. 28, p. 788-795
Non-Patent Literature 5: Journal of Natural Products, 1992, vol. 55, p. 1588-1594
Non-Patent Literature 6: Tetrahedron Letters, 1995, vol. 36, p. 7471-7474
Non-Patent Literature 7: Journal of American Chemical Society, 2002, vol. 124, p. 14556-14557
Non-Patent Literature 8: Angewandte Chemie International Edition, 2007, vol. 46, p. 2254-2256
Non-Patent Literature 9: Journal of Chemical Society Perkin Transaction I, 1995, p. 2345-2353

### DISCLOSURE OF THE INVENTION

### PROBLEMS TO BE SOLVED BY THE INVENTION

An object of the present invention is to provide a proliferation promoting agent for neural stem cells, which comprises a compound produced by *Penicillium* sp. Cud1007, a related compound thereof, or a pharmaceutically acceptable salt thereof, as an active ingredient, and the like; and a compound produced by *Penicillium* sp. CND1007 or a pharmaceutically acceptable salt thereof, which is useful as a proliferation promoting agent for neural stem cells.

### MEANS FOR SOLVING THE PROBLEMS

The present invention relates to the following (1) to (21).
(1) A proliferation promoting agent for neural stem cells, which comprises a compound produced by *Penicillium* sp. CND1007 or a pharmaceutically acceptable salt thereof, as an active ingredient.
(2) The proliferation promoting agent for neural stem cells according to (1), wherein the compound produced by *Penicillium* sp. CND1007 is a compound represented by any one of the following formulae (A) to (BB).

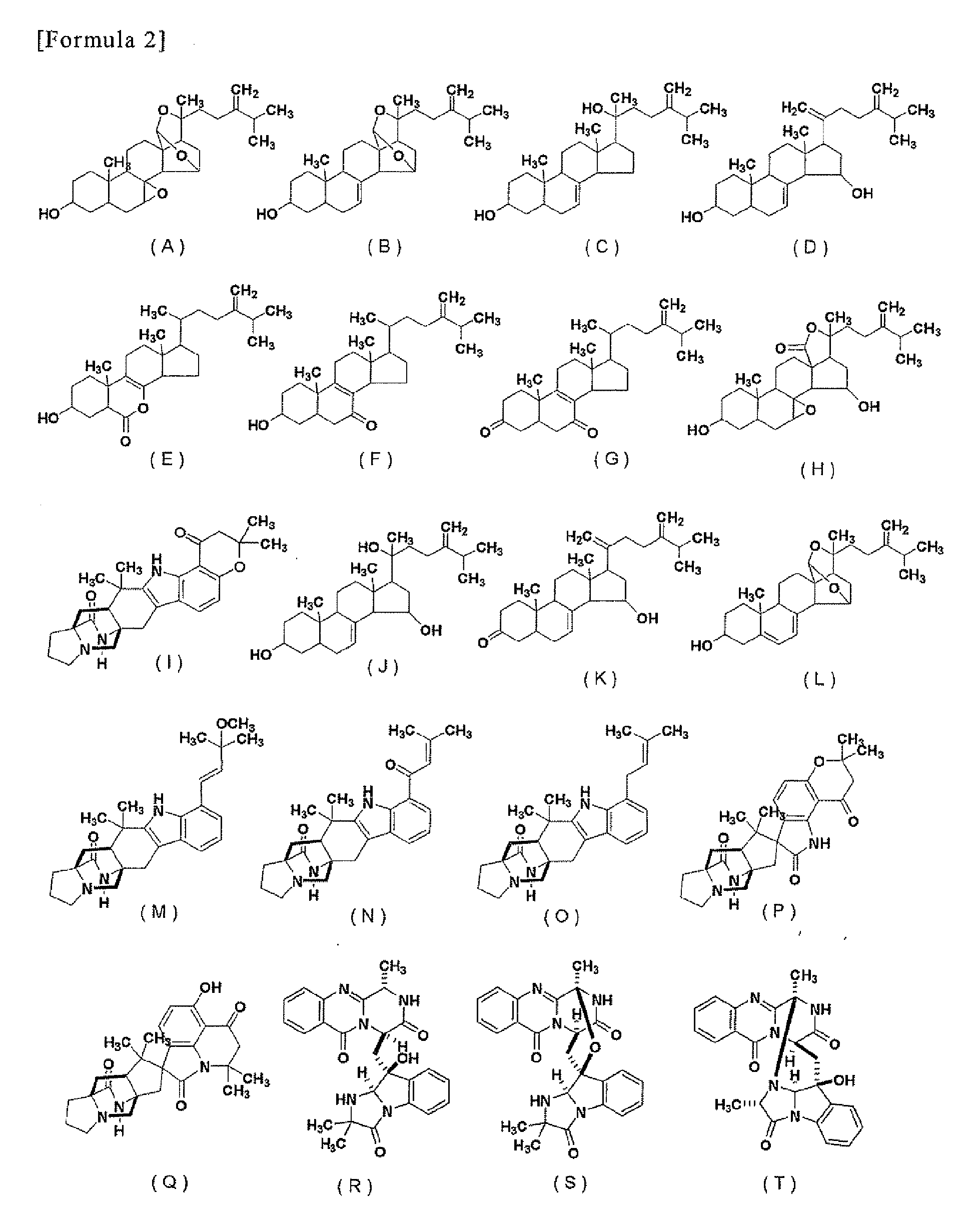

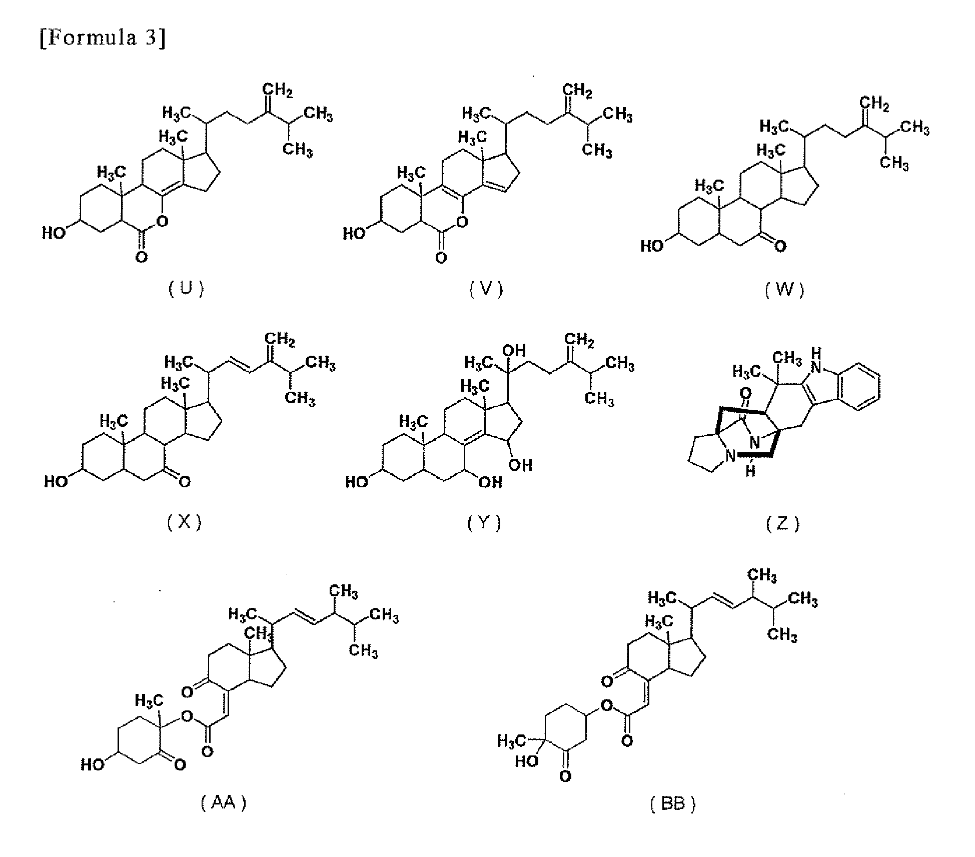

(3) A proliferation promoting agent for neural stem cells, which comprises a compound represented by any one of the following formulae (A1) to (A15) or a pharmaceutically acceptable salt thereof, as an active ingredient.

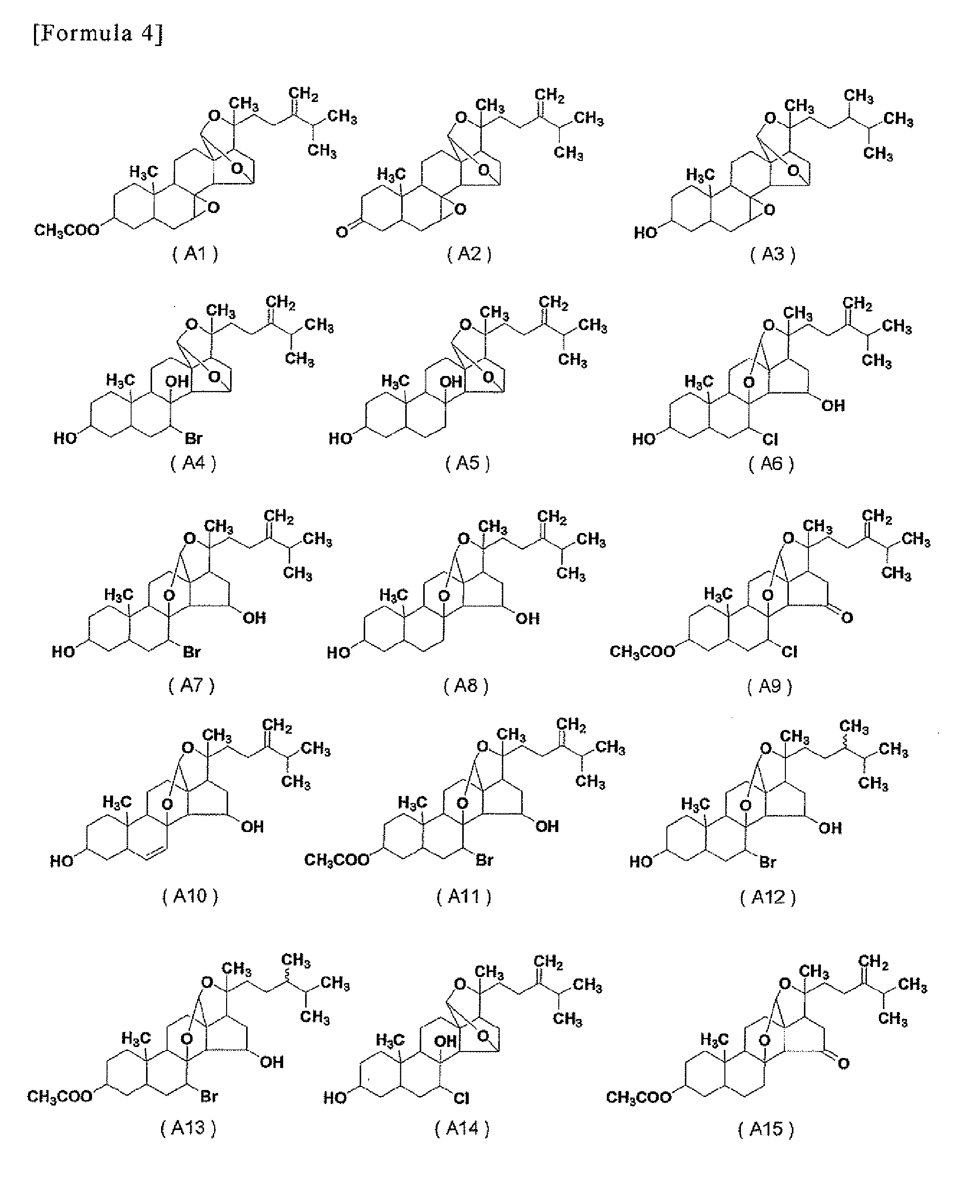

(4) A method for producing neural stem cells, which comprises culturing a neural stem cells to proliferate the neural stem cells in the presence of the compound described in any one of the above-mentioned (1) to (3) or a pharmaceutically acceptable salt thereof, and harvesting the neural stem cells from the culture.
(5) A method for producing neurons, which comprises:
   a step of culturing a neural stem cells to proliferate the neural stem cells in the presence of the compound described in any one of the above-mentioned (1) to (3) or a pharmaceutically acceptable salt thereof, and harvesting the neural stem cells from the culture, and
   a step of culturing the harvested neural stem cells to differentiate them into neurons, and harvesting the neurons from the culture.
(6) A method for producing glial cells, which comprises:
   a step of culturing a neural stem cells to proliferate the neural stem cells in the presence of the compound described in any one of the above-mentioned (1) to (3) or a pharmaceutically acceptable salt thereof, and harvesting the neural stem cells from the culture, and
   a step of culturing the harvested neural stem cells to differentiate them into glial cells, and harvesting the glial cells from the culture.
(7) A compound of any one of the following formulae (B) to (BB) or a pharmaceutically acceptable salt thereof.

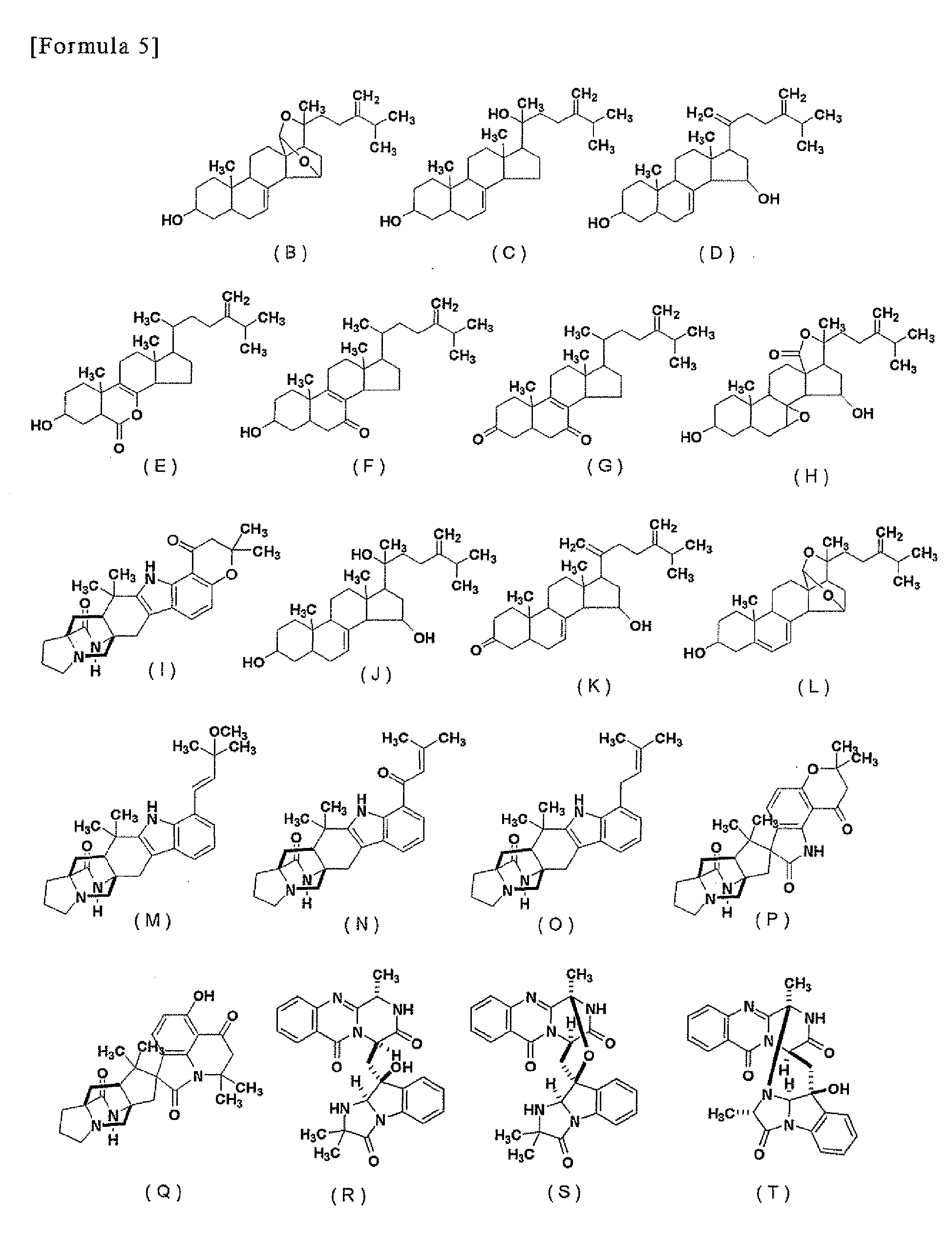

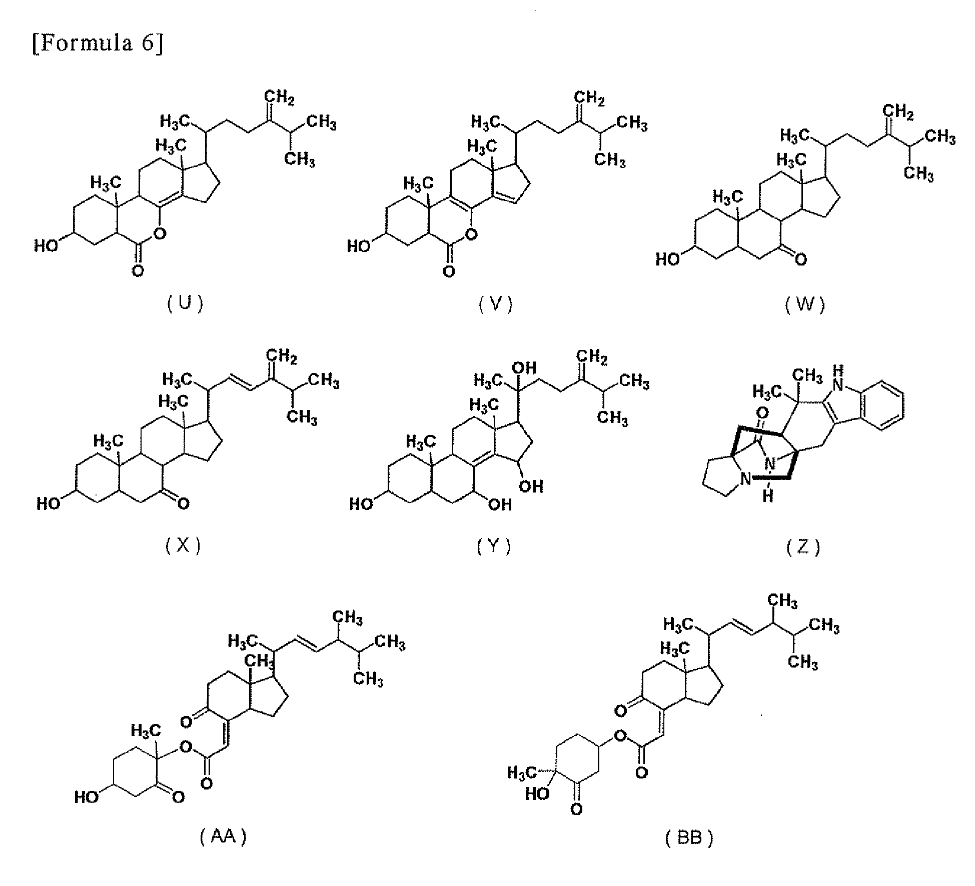

(8) A method for promoting proliferation of neural stem cells, which comprises allowing neural stem cells to contact with a compound produced by *Penicillium* sp. CND1007 or a pharmaceutically acceptable salt thereof.
(9) The method according to (8), wherein the compound produced by *Penicillium* sp. CND1007 is a compound represented by any one of the formulae (A) to (BB) described in (2).
(10) A method for promoting proliferation of neural stem cells, which comprises allowing neural stem cells to contact with the compound represented by any one of the formulae (A1) to (A15) described in (3) or a pharmaceutically acceptable salt thereof.
(11) Use of a compound produced by *Penicillium* sp. CND1007 or a pharmaceutically acceptable salt thereof for the manufacture of a proliferation promoting agent for neural stem cells.
(12) The use according to (11), wherein the compound produced by *Penicillium* sp. CND1007 is a compound represented by any one of the formulae (A) to (BB) described in (2).
(13) Use of the compound represented by any one of the formulae (A1) to (A15) described in (3) or a pharmaceutically acceptable salt thereof for the manufacture of a proliferation promoting agent for neural stem cells.
(14) A compound represented by the following formulae (C), (D), (E), (F), (J), (U), (V), (W), (X) or (Y) or a pharmaceutically acceptable salt thereof.

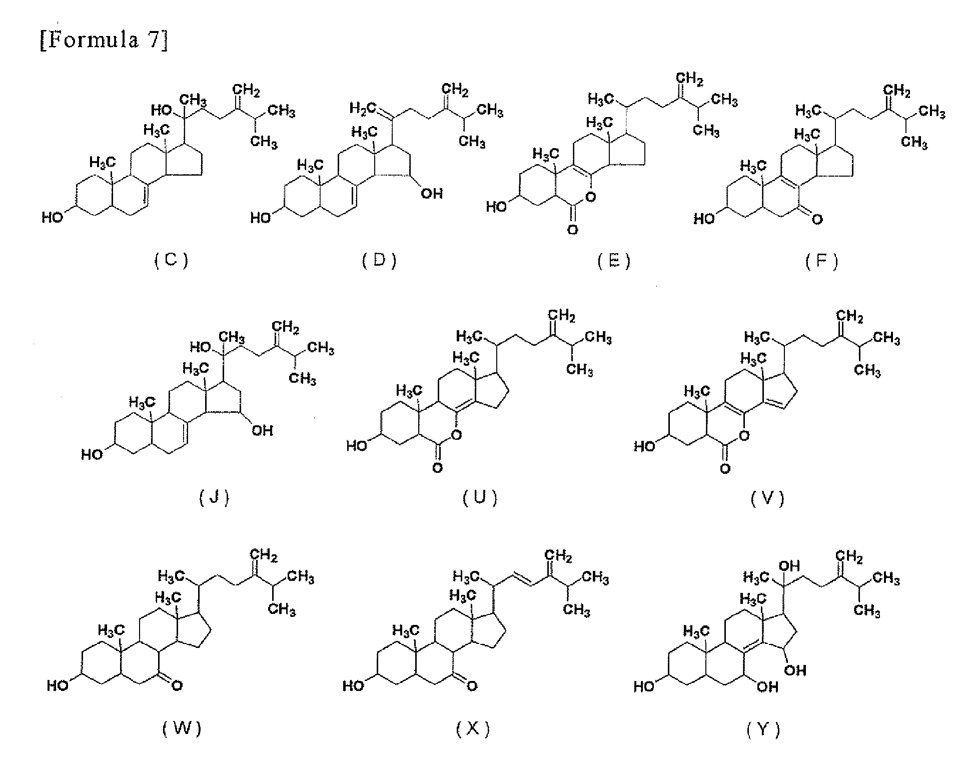

(15) A proliferation promoting agent for neural stem cells, which comprises the compound described in (14) or a pharmaceutically acceptable salt thereof as an active ingredient.
(16) A proliferation promoting agent for neural stem cells, which comprises a compound represented by any one of the following formulae (A1) to (A3) and (A5) or a pharmaceutically acceptable salt thereof as an active ingredient.

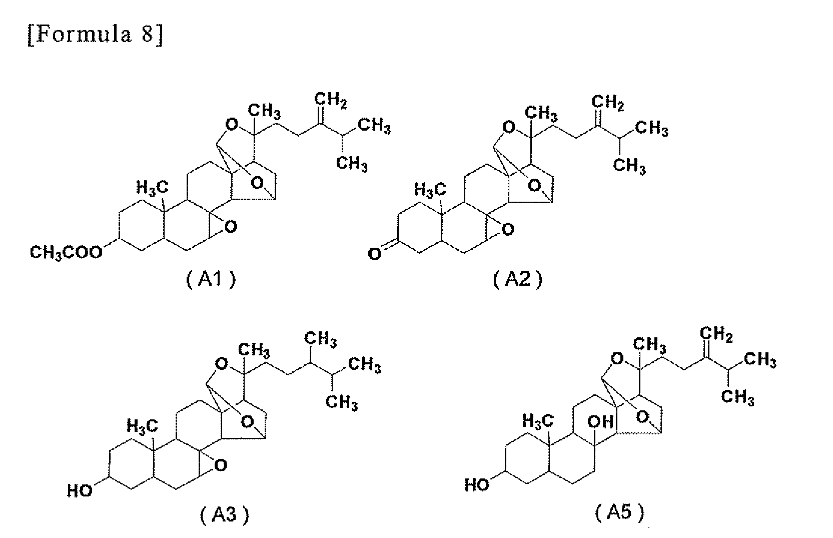

(17) A method for producing neural stem cells, which comprises culturing a neural stem cells to proliferate the neural stem cells in the presence of the compound described in (14) or (16) or a pharmaceutically acceptable salt thereof, and harvesting the neural stem cells from the culture.
(18) A method for producing neurons, which comprises:
   a step of culturing a neural stem cells to proliferate the neural stem cells in the presence of the compound described in (14) or (16) or a pharmaceutically acceptable salt thereof, and harvesting the neural stem cells from the culture, and
   a step of culturing the harvested neural stem cells to differentiate them into neurons, and harvesting the neurons from the culture.
(19) A method for producing glial cells, which comprises:
   a step of culturing a neural stem cells to proliferate the neural stem cells in the presence of the compound described in (14) or (16) or a pharmaceutically acceptable salt thereof, and harvesting the neural stem cells from the culture, and
   a step of culturing the harvested neural stem cells to differentiate them into glial cells, and harvesting the glial cells from the culture.
(20) A method for promoting proliferation of neural stem cells, which comprises allowing neural stem cells to contact with the compound described in (14) or (16) or a pharmaceutically acceptable salt thereof.
(21) Use of the compound described in (14) or (16) or a pharmaceutically acceptable salt thereof for the manufacture of a proliferation promoting agent for neural stem cells.

### EFFECT OF THE INVENTION

The present invention provides a proliferation promoting agent for neural stem cells, which comprises a compound produced by *Penicillium* sp. CND1007, a related compound thereof, or a pharmaceutically acceptable salt thereof, as an active ingredient, and the like, and a compound produced by *Penicillium* sp. CND1007 or a pharmaceutically acceptable salt thereof, which is useful as a proliferation promoting agent for neural stem cells.

### BEST MODE FOR CARRYING OUT THE INVENTION

Hereinafter, the compound represented by formula (A) is referred to as compound (A). The compounds of other formula numbers are similar.
Pharmaceutically acceptable salts of the compounds of the present invention include, for example, pharmaceutically acceptable acid addition salts, metal salts, ammonium salts, organic amine addition salts, amino acid addition salts and the like. Examples of the pharmaceutically acceptable acid addition salts of the compounds of the present invention include inorganic acid salts such as hydrochloride, hydrobromide, nitrate, sulfate and phosphate; organic acid salts such as acetate, oxalate, maleate, fumarate, citrate, benzoate and methanesulfonate; and the like. Examples of the pharmaceutically acceptable metal salts include alkali metal salts such as sodium salt and potassium salt; alkaline earth metal salts such as magnesium salt and calcium salt, aluminum salt, zinc salt and the like. Examples of the pharmaceutically acceptable ammonium salts include salts such as ammonium and tetramethylammonium. Examples of the pharmaceutically acceptable organic amine addition salts include addition salts of morpholine, piperidine and the like. Examples of the pharmaceutically acceptable amino acid addition salts include addition salts of lysine, glycine, phenylalanine, aspartic acid, glutamic acid and the like.

Next, production methods of the compounds of the present invention are described.
Among the compounds of the present invention, the compounds (A) to (BB) are produced by culturing a microorganism which belongs to the genus *Penicillium* and is capable of producing the compounds (A) to (BB), in a medium to form and accumulate the compounds (A) to (BB) in the culture, and recovering the compounds (A) to (BB) from the culture.

As the microorganism which is capable of producing the compounds (A) to (BB), any strain can be used as long as it is a strain which belongs to the genus *Penicillium* and is capable of producing the compounds (A) to (BB). Additionally, mutant strains obtained by mutating these strains by an artificial mutation method such as an ultraviolet ray, X-ray or mutagen treatment or spontaneously mutated mutant strains can also be used in the present invention as long as they are capable of producing compounds (A) to (BB). Specifically, for example, *Penicillium* sp. CND1007 can be cited.

It has been found that the *Penicillium* sp. CND1007 which is isolated from soil, belongs to the genus *Penicillium* of Fungi Imperfecti. It has been deposited on October 11, 2007, as *Penicillium* sp. CND1007 (FERM BP-10917) in International Patent Organism Depositary, National Institute of Advanced Industrial Science and Technology (Central 6, 1-1, Higashi 1-chome, Tsukubashi, Ibaraki, Japan). Its mycological properties are described as follows.

### (I) Macroscopic observation

When it is cultured at 25°C using a Czapek yeast agar medium, diameter of its colony reaches 30 to 35 mm on the 7th day of the culturing. Radial cracks are found on the colony surface which shows grayish green. A yellow percolate is produced on its central part. Also, backside of the colony shows light yellow. A yellow pigment is secreted in the agar medium. When it is cultured at 25°C using a malt extract agar medium, diameter of the colony reaches 35 to 40 mm on the 7th day of the culturing. Surface of the colony is flat and shows light ash green. Additionally, backside of the colony shows dark reddish yellow.

### (II) Microscopic observation

Each hypha has septum and thoroughly branches. The conidiophore is separately formed from the substrate mycelium or aerial mycelium. Conidiophore is smooth, has septum and is 250 to 500 µm in length and 2.5 to 3.0 µm in width. From 3 to 5 metulae are formed on the tip of the conidiophore, and 4 to 7 phialides are formed on the tip of each metula. The metula is colorless, smooth, a single cell, rectangular, 10 to 12.5 µm in length and 3.0 to 4.0 µm in width. The phialide is colorless, smooth, a single cell, ampoule-shaped, 7 to 8 µm in length and 2 to 2.5 µm in width. Conidium is formed from the tip of phialide in a large number by an endogenous budding type mode. The conidium is a single cell and spherical to sub-spherical, shows smooth or minute thorny appearance and is 2.5 to 3.0 µm in diameter. Only the above-mentioned anamorph was observed in this strain, and teleomorph was not observed.

Based on the above mycological properties, regarding its taxonomical position, this strain belongs to the genus *Penicillium* of Hyphomycetes in accordance with The Genera of Fungi Sporulating in Pure Culture, 2nd ed., Cramer Vaduz, J.A. Von Arx, 1974.
In culturing a production strain of the compounds of the present invention, a general filamentous fungi culturing method is used. As the medium, both of a synthetic medium and a natural medium can be use as long as it is a medium which properly contains a carbon source, a nitrogen source, inorganic salts and the like which can be assimilated by the microorganism.

As the carbon source, glucose, starch, dextrin, mannose, fructose, sucrose, lactose, xylose, arabinose, mannitol, molasses and the like can be used alone or in a combination. Additionally, depending on the assimilation ability of the strain, a hydrocarbon, alcohols, organic acid and the like can also be used.
As the nitrogen source, ammonium chloride, ammonium nitrate, ammonium sulfate, sodium nitrate, urea, peptone, meat extract, yeast extract, dry yeast, corn steep liquor (CSL), soybean powder, casamino acid and the like can be used alone or in a combination.

In addition, inorganic salts such as sodium chloride, potassium chloride, magnesium sulfate, calcium carbonate, potassium dihydrogenphosphate, magnesium phosphate octa-hydrate, ferrous sulfate, calcium chloride, manganese sulfate, zinc sulfate and copper sulfate can be added, if necessary. Furthermore, a component which promotes growth of the strain to be used and production of the compound of the present invention (e.g., biosynthesis intermediates mevalonic acid, lanosterol and the like) can be optionally added.

Examples of the culturing method include a liquid culturing method, and an submerged agitation culture method is suitable. The culturing is carried out at a temperature of from 16 to 37°C, preferably from 25 to 32°C and at a pH of from 4 to 10, preferably a pH of from 6 to 8. Aqueous ammonia, an ammonium carbonate solution and the like are used for adjusting pH of the medium. The culturing is completed generally by 1 to 10 days, and it is desirable to stop the culturing when a compound of the present invention is formed and accumulated in the culture (in the culture liquid and cells) and the formed amount in the culture reaches maximum.

Examples of the method for isolating and purifying the compound of the present invention accumulated in the culture liquid from the culture liquid include a method which is generally used for isolating and purifying a general microbial metabolic product from the culture liquid. Specifically, the desired compound is extracted by adding methanol, ethanol, 2-propanol, acetone or the like directly to the culture, or the desired compound is extracted by carrying out a two layer partition extraction with 2-butanone, tert-butanol, n-butanol and the like. Alternatively, the culture is separated into culture filtrate and mycelia by filtration, and the mycelia are further extracted with chloroform, acetone, methanol or the like to obtain the mycelia component. The obtained extract and/or culture filtrate is applied to a column charged with polystyrene adsorbent such as Diaion HP-20, HP-20ss (manufactured by Mitsubishi Chemical Corp.) or the like to adsorb of the desired compound, followed by elution with methanol, acetone or the like. Thereafter, the compound of the present invention can be obtained by isolating and purifying by gel filtration which uses Sephadex LH-20, TOYOPEARL HW 40 or the like or column chromatography which uses octadecyl group type silica gel (ODS), high performance liquid chromatography, silica gel column chromatography or the like.

Additionally, the compound (A) can also be obtained by the methods described for example in Japanese Published Examined Patent Application No. 79080/93, The Journal of Antibiotics, 1988, vol. 41, p. 409 - 410, and the like.
Furthermore, the compounds (A1) to (A15) which are the derivatives thereof can be obtained in accordance with the method described for example in Japanese Published Examined Patent Application No. 5909/96 and the like.

Stereoisomers such as geometrical isomers and optical isomers, tautomers and the like can be present in certain compounds of the present invention. The proliferation promoting agent for neural stem cells of the present invention includes all of the possible isomers and mixtures thereof including them.
When it is desirable to obtain a salt of a compound of present invention, the compound of the present invention may be purified as such when it can be obtained in the form of a salt, and when it is obtained in its free form, a salt of the compound of the present invention may be formed by dissolving or suspending it in an appropriate solvent and adding an acid or base thereto for isolation and purification by formation of salts.

Additionally, the compounds of the present invention and pharmaceutically acceptable salts thereof may be present sometimes in the form of their addition products with water or various solvents, and these addition products are also included in the present invention.
The proliferation promoting agent for neural stem cells of the present invention contains a substance which enhances proliferation of neural stem cells. Examples of the substance which enhances proliferation of neural stem cells include a compound produced by *Penicillium* sp. CND1007 and the like, and they can be obtained by the above-mentioned method. More specifically, examples include the above-mentioned compounds (A) to (BB), (A1) to (A15) and the like.

Specific examples of the compounds of the present invention and the compounds contained in the proliferation promoting agent of the present invention for neural stem cells are shown in Table 1 to Table 5. However, the compounds of the present invention and the like are not limited thereto.

### [Table 1]

**Table 1**

| Example No. | Compound No. | | Example No. | Compound No. | |
|---|---|---|---|---|---|
| 1 | B | 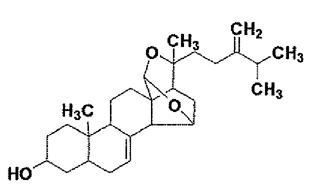 | 1 | C | 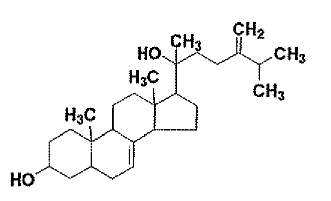 |
| 1 | D | 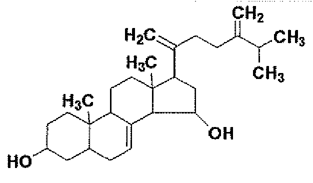 | 1 | E | 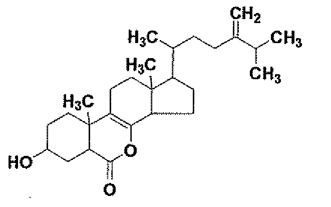 |
| 1 | F | 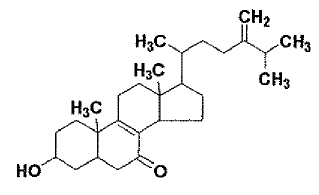 | 1 | G | 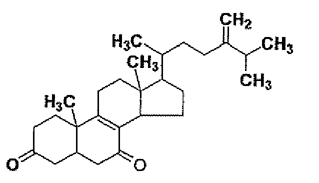 |
| 2 | H | 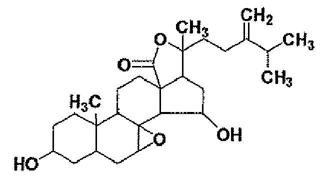 | 1 | I | 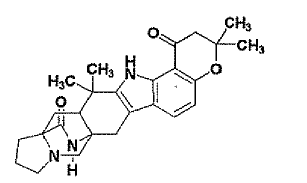 |
| 2 | J | 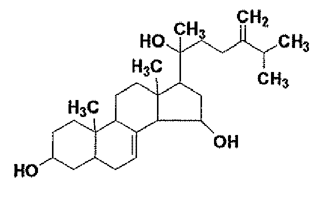 | 1 | K | 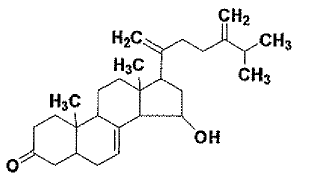 |

### [Table 2]

**Table 2**

| Example No. | Compound No. | | Example No. | Compound No. | |
|---|---|---|---|---|---|
| 1 | L | 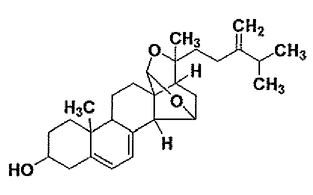 | 1 | M | 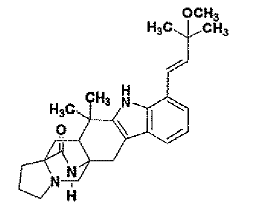 |
| 1 | N | 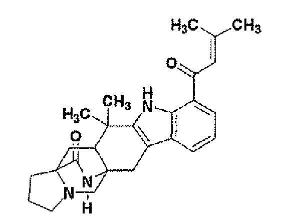 | 1 | O | 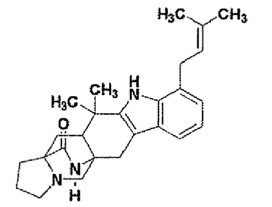 |
| 1 | P | 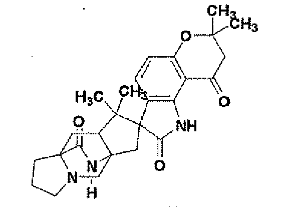 | 1 | Q | 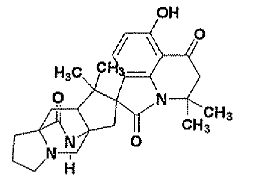 |
| 1 | R | 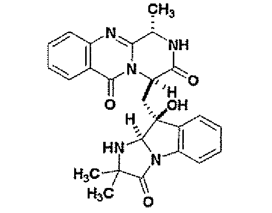 | 1 | S | 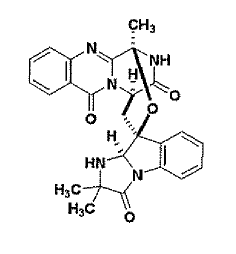 |
| 1 | T | 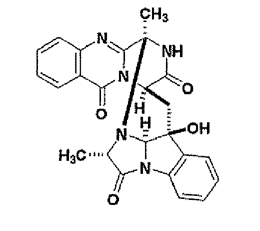 | | | |

### [Table 3]

**Table 3**

| Example No. | Compound No. | | Example No. | Compound No. | |
|---|---|---|---|---|---|
| 3 | U | 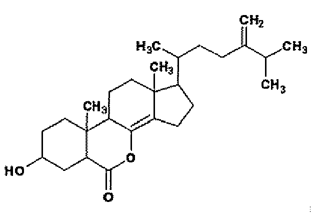 | 3 | V | 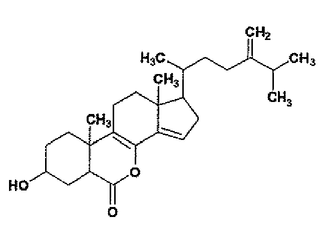 |
| 3 | w | 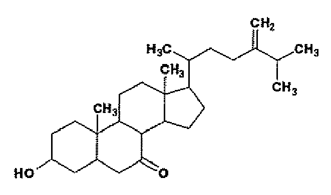 | 3 | X | 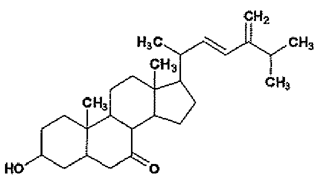 |
| 4 | Y | 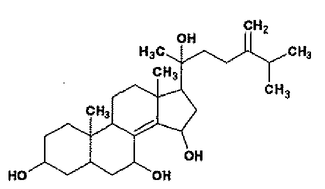 | 4 | Z | 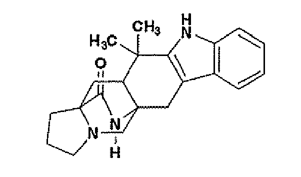 |
| 4 | AA | 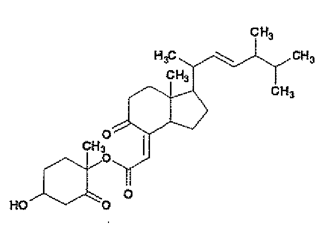 | 4 | BB | 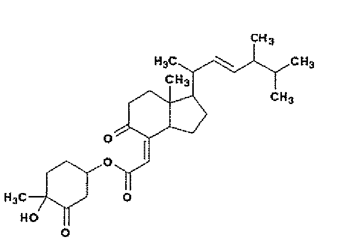 |

### [Table 4]

**Table 4**

| Reference Example No. | Compound No. | | Reference Example No, | Compound No. | |
|---|---|---|---|---|---|
| 2 | A1 | 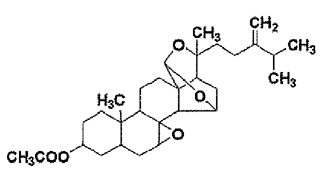 | 2 | A2 | 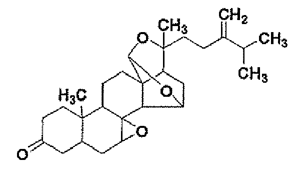 |
| 2 | A3 | 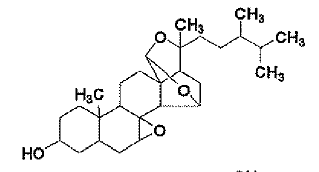 | 2 | A4 | 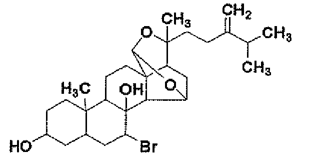 |
| 2 | A5 | 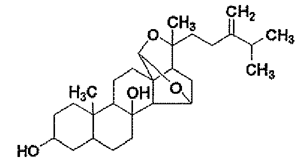 | 2 | A6 | 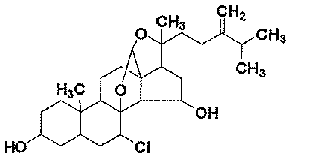 |
| 2 | A7 | 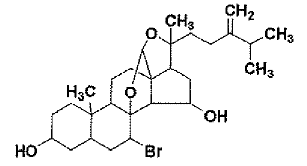 | 2 | A8 | 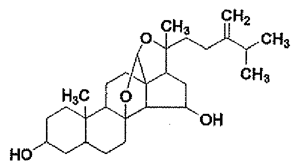 |
| 2 | A9 | 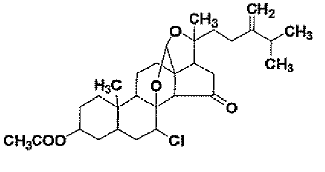 | 2 | A10 | 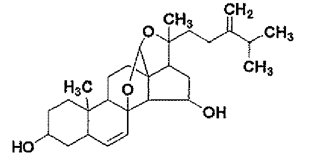 |

### [Table 5]

**Table 5**

| Reference Example No. | Compound No. | | Reference Example No. | Compound No. | |
|---|---|---|---|---|---|
| 2 | A11 | 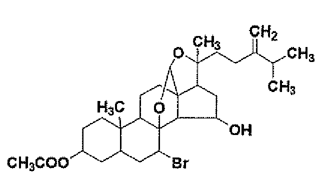 | 2 | A12 | 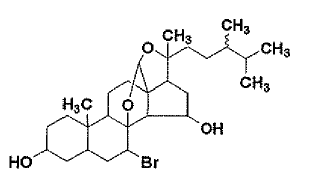 |
| 2 | A13 | 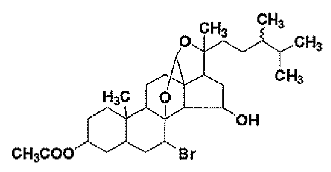 | 2 | A14 | 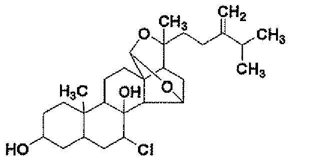 |
| 2 | A15 | 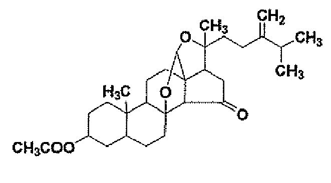 | 3 | A | 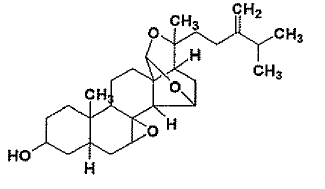 |

Among the compounds shown in the above-mentioned Table 1 to Table 5, for example, a compound which is produced by *Penicillium* sp. CUD 1007 and has the basic structure represented by the following formula (α) as its chemical structure is preferable. A compound having hydroxy on the ring A moiety of the following formula (α) is more preferable.

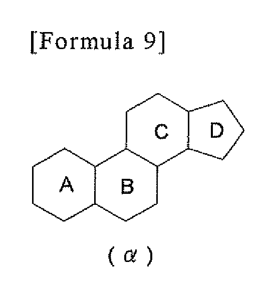

More specifically, for example, compounds A, B, C, D, E, F, H, J, L, U, V, W, X and the like are preferable, and compounds C, D, E, F, U, V, W, X and the like are more preferable.
Next, the proliferation promoting agent for neural stem cells and the production method of neural stem cells of the present invention are explained.
When the proliferation promoting agent for neural stem cells of the present invention is allowed to contact with neural stem cells *in vitro,* it can promote proliferation of the neural stem cells.

The stem cell is a cell possessing a pluripotency which is an ability to differentiate into a variety of cells and an ability to self-renew of new stem cells by symmetric or asymmetric division. On the other hand, a cell which enters a certain lineage and is destined to carry through its differentiation after a limited division is called progenitor cells. However, since it is difficult to strictly distinguish neural stem cells and neural progenitor cells or glial progenitor cells, neural stem cells referred as in the present application include neural progenitor cells and glial progenitor cells.

Although the neural stem cell is not particularly limited, cerebral adult neural stem cells are preferable.
Although the brain may be the brain of any animal, the brain of a mammal is preferable and rat, mouse, monkey, human or the like is more preferable.
Examples of the method for preparing adult neural stem cells from an animal include a method in which a cerebral cell crude extract is prepared by extracting the brain from an adult animal by a surgical means and the adult stem cells are concentrated from the crude extract, in accordance with the methods described, for example, in The Journal of Neuroscience, 1999, vol. 19, p. 8487 - 8497, Genes and Development, 1996, vol. 10, p. 3129 - 3140, and the like.

Additionally, examples of the method for preparing adult neural stems cells from human include a method in which a cerebral cell crude extract is prepared by collecting a tissue from the lateral ventricle wall of a patient of neurological disorder by biopsy and the adult stem cells are concentrated from the crude extract, in accordance with the method described in Experimental Cell Research, 2003, vol. 289, p. 378 - 383.
The proliferation promoting agent for neural stem cells of the present invention can be used in a method for producing neural tem cells, wherein proliferation of neural stem cells is promoted by allowing it to contact with the neural stem cells *in vitro* and culturing the neural stem cells, and the neural stem cells are harvested from the culture.

When the proliferation promoting agent for neural stem cells of the present invention is used *in vitro,* it is preferable to use a compound of the present invention having the activity to promote proliferation of neural stem cells, or a pharmaceutically acceptable salt thereof, by dissolving it in a solution which can dissolve the compound or a pharmaceutically acceptable salt thereof. Examples of the solution include water, dimethyl sulfoxide (DMSO) and the like. Additionally, it can also be used by dissolving in various buffers such as phosphate buffered saline (PBS).

When adult neural stem cells are cultured in the presence of the proliferation promoting agent for neural stem cells of the present invention, it is preferable to add the proliferation promoting agent for neural stem cells at a concentration of 1 pmol/L to 1 mmol/L, based on approximately 6.25×10⁴ cells/cm² of the adult neural stem cells for reaction. Proliferation of neural stem cells can be promoted by allowing adult neural stem cells to contact with the proliferation promoting agent for neural stem cells of the present invention, followed by static culturing at 37°C for 1 to 14 days under an atmosphere of 5% CO₂ while exchanging the whole volume or a partial volume of the medium at intervals of 2 days.

The medium may be any medium as long as it is a medium which does not obstruct proliferation promotion of neural stem cells. For example, it is preferable to use DMEM/F12 medium (manufactured by Invitrogen) containing 1% N-2 additives (manufactured by Invitrogen), and the like.
Additionally, the neural stem cells prepared by the above-mentioned culturing can be differentiated into neurons, by carrying out static culturing at 37°C for 1 to 14 days under an atmosphere of 5% CO₂, while exchanging the whole volume or a partial volume of a medium at intervals of 2 days, in a medium which does not contain the proliferation promoting agent for neural stem cells of the present invention but contains, for example, 1 nmol/L to 1 mmol/L of all trans retinoic acid, 1 nmol/L to 1 mmol/L of forskolin or 0.1 ng/mL to 1 mg/mL of platelet-derived growth factor (PDGF), or the like.

The medium may be any medium as long as it is a medium which does not obstruct differentiation into neurons. For example, it is preferable to use DMEM/F12 medium (manufactured by Invitrogen) containing 1% N-2 additives (manufactured by Invitrogen), and the like.
Additionally, the neural stem cells prepared by the above-mentioned culturing can be differentiated into glial cells, by carrying out static culturing at 37°C for 1 to 14 days under an atmosphere of 5% CO₂, while exchanging the whole volume or a partial volume of a medium at intervals of 2 days, in a medium which does not contain the proliferation promoting agent for neural stem cells of the present invention but contains, for example, 0.1 ng/mL to 1 mg/mL of leukemia inhibitory factor (LIF), 0.1 ng/mL to 1 mg/mL of a bone formation factor-2 (BMP-2), or the like.

The medium may be any medium as long as it is a medium which does not obstruct differentiation into glial cells. For example, it is preferable to use DMEM/F12 medium (manufactured by Invitrogen) containing 1% N-2 additives (manufactured by Invitrogen), and the like.
The neural stem cells, neuron or glial cells which are prepared by the above-mentioned culturing can be used in the treatment of a neurological disorder, by harvesting them from the medium and transplanting them into the affected region of a patient of the neurological disorder. The neurological disorder include, for example, Parkinson's disease, Alzheimer's disease, Down syndrome, cerebrovascular accidents, stroke, spinal cord injury, triplet repeat disease, multiple sclerosis, amyotrophic lateral sclerosis, polyneuropathy, epilepsy, anxiety disorder, schizophrenia, depression, bipolar disorder and the like.

Next, proliferation promoting activity of typical compounds is specifically described based on test examples. The following test examples are provided for the exemplification purpose only. Accordingly, the scope of the present invention is not limited to the following test examples.

### Test Example 1: Proliferation promoting activity on neural stem cells (1)

Rat adult neural stem cell line ANSC-7 cells prepared by the method described in the following Reference Example 1 were suspended in DMEM/F12 medium (manufactured by Invitrogen) supplemented with N-2 additives [5 µg/mL insulin (manufactured by Sigma), 100 µg/mL bovine apotransferrin (manufactured by Sigma), 6.3 ng/mL progesterone (manufactured by Sigma), 1.6 µg/mL putrescine (manufactured by Sigma) and 5.2 ng/mL sodium selenate (manufactured by Sigma)] and an antibiotics mixed liquid [0.05 U/mL penicillin and 0.05 µg/mL streptomycin (manufactured by Invitrogen)] (to be referred to as assay medium hereinafter), at a density of 1.7 ×10⁵ cells/mL; inoculated at 0.1 mL in a 96 well plate (manufactured by Costar) of which surface was processed with polyornithine and laminin; and cultured overnight at 37°C under an atmosphere of 5% CO₂. Thereafter, 50 µl of the culture supernatant was removed, and 50 µl of a test compound which was serially diluted with the assay medium to 2 times of the final concentration or DMSO (negative control) was added to each well. After the culturing for 96 hours, 50 µl of the culture supernatant was removed, and 50 µl of a 15% neutral buffered formalin liquid (manufactured by Wako Pure Chemical Industries, Ltd.) cooled to be 4°C was added to each well and allowed to stand for 1 hour. Thereafter, by washing twice using PBS containing 0.3% Triton X-100 (manufactured by Nacalai Tesque) (hereinafter TBST), 50 µl of Hoechst 33342 (manufactured by Nacalai Tesque) adjusted to 3.3 µmol/L with 5% skim milk solution diluted with PBS containing 0.5% Triton X-100 was added to each well and the nucleus was stained overnight at 4°C in the dark. After washing twice with 100 µl/well of TBST and then once with PBS and subsequently soaking in 100 µl/well of PBS, fluorescence intensity of the nucleus-stained cells was measured using a fluorometer FDSS 6000 (Hamamatsu Photonics K.K.). By regarding the measured value of well to which the cells were not inoculated as 0%, and the measured value of negative control as 100%, relative values in the test compound addition group were calculated.

Each of the compounds A, C and E showed a proliferation promoting activity of 150% or more at 0.32 µmol/L. Also, each of the compounds A1, A2, A3 and A5 showed a proliferation promoting activity of 160% or more at 0.32 µmol/L. Additionally, each of the compounds B, D and F showed a proliferation promoting activity of 140% or more at 1.0 µmol/L.

### Test Example 2: Proliferation promoting activity on neural stem cells (2)

Rat adult neural tem cell line ANSC-7 cells prepared by the method described in the following Reference Example 1 were suspended in the assay medium at a density of 1.7×10⁵ cells/mL; inoculated in 0.1 mL portion in a 96 well plate (manufactured by Costar) of which surface was processed with polyornithine and laminin; and cultured overnight at 37°C under an atmosphere of 5% CO₂. Thereafter, 50 µl of the culture supernatant was removed, and 50 µl of a test compound which was serially diluted with the assay medium to 2 times of the final concentration or DMSO (negative control) was added to each well. After the culturing for 96 hours, 10 µl per well of a viable cell counting reagent SF (manufactured by Nacalai Tesque) was added to the culture. After culturing at 37°C for 3 hours in a 5% CO₂ incubator and subsequent stirring for 1 minute, absorbance at 490 nm (control wavelength 655 nm) was measured using a microplate spectrophotometer Emax (manufactured by Molecular Device). By regarding the measured value of well to which the cells were not inoculated as 0%, and the measured value of negative control as 100%, relative values in the test compound addition group were calculated.

Each of the compounds A, C, E and L showed a proliferation promoting activity of 130% or more at 0.32 µmol/L. Also, each of the compounds D, F, H and K showed a proliferation promoting activity of 130% or more at 1.0 µmol/L. Additionally, the compound J showed a proliferation promoting activity of 125% or more at 3.2 µmol/L.

### Test Example 3: Proliferation promoting activity on neural stem cells (3)

Each of the tests was carried out in the same manner as in Test Example 2. Each of the compounds U and V showed a proliferation promoting activity of 120% or more at 1 nmol/L. Each of the compounds W and X showed a proliferation promoting activity of 120% or more at 10 nmol/L. Also, the compound M showed a proliferation promoting activity of 120% at 0.32 µmol/L. The compound N showed a proliferation promoting activity of 120% at 1 µmol/L. In addition, each of the compounds Y, AA and BB showed a proliferation promoting activity of 120% at 3.2 µmol/L.

### [Reference Example 1] Isolation and culturing of adult neural stems cells from rat brain

After putting a 7-week-old Sprague Dawley Rat to sleep by ether anesthesia and subsequent decapitation, the skull was cut open from the parietal region to extract the brain. Under a microscope, tissues including circumventricular region were isolated from the extracted brain by using ophthalmic scissors and tweezers. The tissues including circumventricular region were cut into fragments of about 1 mm³ using ophthalmic scissors and scalpels and then subjected to 30 minutes of digestion reaction at 37°C in 5 mL of Hanks' buffer containing 2.5 U/mL of papain, 250 U/mL of DNase (all manufactured by Worthington, Freehold, NJ) and 1 U/mL of a neutral protease (Dispase, manufactured by Boehringer-Mannheim Corp.) (HBSS buffer, manufactured by Invitrogen). The mixture of cells and tissues obtained by the reaction was washed three times with DMEM (manufactured by Invitrogen) containing 10% fetal bovine serum (manufactured by Hyclone) and then dissolved in the DMEM containing 10% fetal bovine serum, followed by removing the undigested materials using a nylon mesh of 10 µm.

The thus obtained cells crude extract was cultured overnight on a culture dish of 10 cm, in an incubator of 37°C using DMEM/F12 medium (manufactured by Invitrogen) containing 10% fetal bovine serum. On the next day, the medium was replaced with DMEM/F12 containing 1% of N-2 additives (manufactured by Invitrogen) and 20 ng/mL of FGF 2 (manufactured by Pepro Tech) and the culturing was started. Once in 3 days, half of the medium was replaced with new DMEM/F12 containing 1% of N-2 additives and 20 ng/mL of FGF 2 and the culturing was continued.

When a small colony consisted of small cells was formed, it was treated with 1% trypsin for 30 seconds to 1 minute, and the cells detached were harvested. The harvested cells were inoculated on a multiple well culture dish (manufactured by Fisher Scientific) which had been coated at room temperature overnight using 10 µg/mL of polyornithine (manufactured by Sigma) and at 37°C overnight using 5 µg/mL of mouse EHS tumor-derived laminin (Becton Dickinson), and the culturing was continued.

By continuing the above-mentioned culturing, small cells having small protrusion and thickness were concentrated. The cells were used as adult neural stem cells in the above-mentioned tests (Test Examples 1 to 3).
Although the present invention is explained in more detail based on examples and reference examples, the scope of the present invention is not limited to these examples.
In this connection, physicochemical data on each compound in the following examples were measured by the following equipments. In the case of the proton nuclear magnetic resonance, an exchangeable proton cannot be observed clearly in some cases depending on the compounds and measuring conditions. As the notation of multiplicity of signal, those which are generally used are used, wherein br represents that it is a broad signal in appearance.

¹H NMR and ¹³C NMR: Bruker DMX 500 (500 and 125 MHz), JEOL Alpha 400 (400 and 100 MHz) or JEOL Lambda 300 (300 and 75 MHz)
FAB-MS: JEOL JMS-HX/HX110A
ESI-MS: Micromass ZMD

### EXAMPLE 1

### Production of compounds A to G and K to T

As the first seed medium and second seed medium, a medium comprising glucose (20 g/L), mashed potatoes (30 g/L) and dry yeast extract (5 g/L) (pH 6.5) was used, and as the main fermentation medium, a medium comprising sucrose (30 g/L), soluble starch (20 g/L), corn steep liquor (CSL) (30 g/L) and calcium carbonate (5 g/L) (pH 5.0) was used. A piece of agar containing *Penicillium* sp. CND1007 was inoculated into the first seed medium (10 mL) which had been added into a 70 mL capacity test tube, followed by shaking culturing at 28°C for 72 hours. Next, 25 mL per flask of the first seed culture liquid was inoculated into the second seed medium (475 mL) which had been added into each of 2 L capacity conical flasks, followed by shaking for 72 hours in the same manner. Subsequently, 900 mL per fermenter of the second seed culture liquid was inoculated into the main fermentation medium (about 54 L) which had been dispensed into three 30 L capacity jar fermenters, followed by agitation culturing (the number of revolutions 250 rpm) at 25°C for 8 days. Additionally, 25 mL per flask of the second seed culture liquid was inoculated into the main fermentation medium (about 10 L) which had been dispensed into 20 conical flasks each having 2 L capacity, followed by agitation culturing (the number of revolutions 220 rpm) at 25°C for 8 days.

A filter aid (Radiolite # 600, manufactured by Showa Chemical Industry) was added at a ratio of 10% by weight to the thus obtained fermentation culture liquid (64 L) and then the culture filtrate and cells were separated by suction filtration. The separated cells were mixed with 15 L of methanol, followed by extraction twice at room temperature. The extract (30 L) was concentrated to 10 L under reduced pressure and applied to a column filled with 2 L of Diaion HP 20 (manufactured by Mitsubishi Chemical Corp.) to adsorb the desired compound. After washing with water, 40% methanol and 70% methanol, the desired compound was eluted with 100% methanol and 30% acetone/methanol. The eluate (6 L) was concentrated to 1 L under reduced pressure and then extracted three times with chloroform (1 L). The residue (15 g) obtained by concentrating the extract under reduced pressure was applied to a column filled with 500 mL of silica gel and eluted stepwise using n-hexane, ethyl acetate, methanol and a mixed solvent thereof. Components contained in each eluate was detected by thin layer chromatography, and eluates containing the same component were combined to obtain fractions of 20 to 40% ethyl acetate/n-hexane elution fraction (fraction 1), 60% ethyl acetate/n-hexane elution fraction (fraction 2), 60 to 80% ethyl acetate/n-hexane elution fraction (fraction 3), 80% ethyl acetate/n-hexane to ethyl acetate elution fraction (fraction 4), ethyl acetate to 25% methanol/ethyl acetate elution fraction (fraction 5) and 25% methanol/ethyl acetate elution fraction (fraction 6).

The fraction 1 (2.3 g) was applied to a column filled with neutral alumina (50 mL), followed by elution and eluted with ethyl acetate to remove contaminating higher fatty acids. The residue (502 mg) obtained by concentrating the eluate was separated and purified by fractional high performance liquid chromatography [column SunFire^{™} Prep C18 OBD 10 µm (manufactured by Waters), φ 19×250 mm, column temperature 40°C, flow rate 10 mL/min, stepwise elution with 85 to 100% methanol aqueous solution] to obtain each of compound C (57.0 mg), compound G (12.6 mg) and compound K (36.0 mg).

The fraction 2 (300 mg) was applied to a column filled with 20 mL of silica gel, followed by elution with n-hexane and ethyl acetate. Fractions containing the compounds of interest were collected and concentrated, and the thus obtained residue (200 mg) was separated and purified by fractional high performance liquid chromatography [column SunFire^{™} Prep C18 OBD 10 µm, φ 19×250 mm, column temperature 40°C, flow rate 10 mL/min, stepwise elution with 85 to 100% methanol aqueous solution] to obtain each of compound L (15.4 mg), compound B (21.1 mg), compound D (40.0 mg) and compound E (44.6 mg).

By separating and purifying the fraction 3 (1.5 g) by fractional high performance liquid chromatography [column SunFire^{™} Prep C18 OBD 10 µm, φ 19×250 mm, column temperature 40°C, flow rate 10 mL/min, stepwise elution with 85 to 100% methanol aqueous solution], each of compound B (9.2 mg) and compound F (23.3 mg) was obtained.
By recrystallizing the fraction 4 (4.95 g) from methanol, the compound A (4.0 g) was obtained.

The fraction 5 (1.17 g) was separated and purified by silica gel column chromatography (methanol/chloroform and methanol/ethyl acetate) and then by fractional high performance liquid chromatography [column SunFire^{™} Prep C18 OBD 10 µm, φ 19×250 mm, column temperature 40°C, flow rate 10 mL/min, stepwise elution with 40 to 100% methanol aqueous solution], to obtain each of compound R (5.3 mg), compound S (4.5 mg) and compound T (10.4 mg).

The fraction 6 (4.01 g) was separated and purified by silica gel column chromatography (methanol/ethyl acetate), alumina column chromatography (ethyl acetate) and fractional high performance liquid chromatography [column SunFire^{™} Prep C18 OBD 10 µm, φ 19×250 mm, column temperature: room temperature, flow rate: 10 mL/min, stepwise elution with 45 to 95% acetonitrile aqueous solution] to obtain each of compound I (113.0 mg), compound M (3.4 mg),compound N (0.7 mg), compound O (2.0 mg), compound P (6.1 mg) and compound Q (7.1 mg).

### EXAMPLE 2

### Production of compounds H and J

A piece of agar containing *Penicillium* sp. CND1007 was inoculated into the first seed medium (10 mL) described in Example 1 which had been put into a 70 mL capacity test tube, followed by shaking culturing at 28°C for 72 hours. Into the second seed medium (10 mL) described in Example 1 which had been put into 70 mL capacity test tubes, 0.5 mL of the first seed liquid medium was inoculated, followed by shaking for 72 hours in the same manner. Into the main fermentation medium (50 mL) described in Example 1 which had been put into 60 flasks of a 300 mL capacity conical flask (3 L in total), 5 mL of the thus obtained second seed culture liquid was inoculated, followed by agitation culturing (the number of revolution 220 rpm) at 25°C for 5 days.

A filter aid was added at a ratio of 10% by weight to the thus obtained fermentation culture liquid (3 L) and the culture filtrate and mycelia were separated by suction filtration. The separated mycelia were mixed with methanol (2 L); followed by thoroughly agitation; and extraction, and then filtration using a suction filtration machine. To extracts, 2 L of methanol/acetone (1/1) was added and extraction step was repeated. The extracts were combined, concentrated under reduced pressure and then applied to a column filled with 450 mL of Diaion HP 20, to adsorb the compounds of interest. After washing with an aqueous solution of 30% methanol (0.9 L) and an aqueous solution of 70% methanol (1.35 L), the compounds of interest were eluted with 1.35 L for each of methanol and methanol/acetone (7/3). Each of the eluates was concentrated under reduced pressure and they were made into 250 mL of an aqueous solution of 80% methanol and 250 mL of an aqueous solution of 70% methanol, repeatedly. They were washed twice with 100 mL of hexane, combined and then made into an aqueous solution of 40% methanol by adding water (900 mL). Next, the thus obtained aqueous solution was extracted with chloroform to obtain 2.21 g of crude extract. The thus obtained crude extract was dissolved in methanol; a small amount of silica gel was added thereto; and methanol was evaporated to adsorb the crude extract to silica gel. The above-mentioned crude extract adsorbed to silica gel was layered over a column filled with 200 mL of silica gel; washed with 500 mL for each of hexane/ethyl acetate (9/1) and chloroform; and then developed with a methanol/chloroform mixed solvent. By eluting fractions with 0.67% methanol and 1.0% methanol, followed by concentration, 600.3 mg of the thus obtained brown substance was dissolved in methanol. Formed crystals were removed, and the resulting mother liquid was separated and purified by fractional high performance liquid chromatography [column SunFire^{™} Prep C18 OBD 10 µm, φ 19×250 mm, column temperature 40°C, flow rate 10 mL/min, elution with methanol/acetonitrile/water (30/40/30)] in the same manner, to obtain compound H (2.1 mg) which was eluted at a retention time of 26.5 minutes. Furthermore, by separating and purifying by fractional high performance liquid chromatography [column SunFire^{™} Prep C18 OBD 10 µm, φ 19×250 mm, column temperature: 40°C, flow rate: 10 mL/min, and elution with methanol/acetonitrile/water (40/40/20)] in the same manner, compound J (1.0 mg) which was eluted at a retention time of 11.5 minutes was obtained.

Physicochemical data of the compounds A to T obtained in the above-mentioned examples 1 and 2 are as follows.
Physicochemical data of compound A
FAB mass spectrum (*m*/*z*): 443 [M+H]⁺, 465 [M+Na]⁺.
¹H-NMR:δ(ppm, CD₃OD) 0.85 (3H, s), 1.00 (1H, ddd, *J* = 13.4, 13.4, 3.7 Hz), 1.03 (3H, d, *J* = 6.8 Hz), 1.03 (3H, d, *J* = 6.8 Hz), 1.15 (1H, m), 1.28 (1H, dddd, *J* = 12.7, 12.7, 5.3, 3.3 Hz), 1.33 (1H, m), 1.35 (3H, s), 1.37 (1H, m), 1.57 (1H, dd, *J* = 15.3, 12.8 Hz), 1.58 (1H, m), 1.60 (1H, m), 1.69 (1H, m), 1.74 (1H, m), 1.75 (2H, m), 1.78 (1H, m), 1.79 (1H, m), 1.85 (1H, m), 1.87 (1H, m), 1.93 (1H, d, *J* = 13.3 Hz), 1.95 (1H, m), 2.00 (1H, m), 2.17 (1H, d, *J* = 9.3 Hz), 2.25 (1H, m), 2.28 (1H, s), 2.32 (1H, m), 3.30 (1H, m), 3.47 (1H, m), 3.93 (1H, br.s), 4.69 (1H, d, *J* = 1.3 Hz), 4.75 (1H, s), 5.36 (1H, s).
¹³C-NMR:δ(ppm, CD₃OD) 13.21, 22.31, 22.31, 22.64, 27.63, 29.19, 29.71, 30.39, 31.43, 34.88, 35.16, 36.07, 38.19, 39.01, 40.69, 41.07, 49.56, 50.14, 55.40, 57.96, 60.04, 62.07, 71.47, 74.54, 86.95, 106.98, 107.96, 157.09.

### Physicochemical data of compound B

FAB mass spectrum (*m*/*z*):427 [M+H]⁺, 409 [M-H₂O+H]⁺.
High resolution FAB mass spectrum (*m*/*z*):
Found value 427.3185
Theoretical value 427.3212 (as C₂₈H₄₃O₃).
¹H-NMR:δ(ppm, CD₃OD) 0.78 (3H, s), 1.04 (3H, d, *J* = 6.8 Hz), 1.04 (3H, d, *J* = 6.9 Hz), 1.12 (1H, ddd, *J* = 13.7, 13.7, 3.6 Hz), 1.25 (1H, m), 1.35 (3H, s), 1.37 (1H, m), 1.39 (1H, m), 1.40 (1H, m), 1.69 (1H, m), 1.71 (1H, m), 1.76 (1H, m), 1.78 (2H, m), 1.78 (2H, m), 1.83 (1H, m), 1.84 (1H, m), 1.84 (1H, m), 1.88 (1H, m), 2.00 (2H, m), 2.01 (1H, m), 2.18 (1H, d, *J* = 9.4 Hz), 2.25 (1H, m), 2.26 (1H, m), 2.32 (1H, br.s), 3.51 (1H, m), 4.40 (1H, s), 4.71 (1H, d, *J* = 1.3 Hz), 4.76 (1H, s), 5.17 (1H, s), 5.59 (1H, m).
¹³C-NMR:δ(ppm, CD₃OD) 13.57, 22.33, 22:33, 24.21, 27.79, 28.17, 30.39,30.60, 32.00, 35.19, 35.19, 35.29, 38.26, 38.48, 41.18, 41.20, 49.77, 49.84, 57.33, 60.40, 71.47, 75.52, 87.64, 106.99, 107.03, 120.45, 135.06, 157.11.

### Physicochemical data of compound C

FAB mass spectrum (*m*/*z*): 437 [M+Na]⁺, 397 [M-H₂O+H]⁺.
High resolution FAB mass spectrum (*mlz)*:
Found value 397.3475
Theoretical value 397.3471 (as C₂₈H₄₅O)
Found value 437.3334
Theoretical value 437.3395 (as C₂₈H₄₆O₂Na)
¹H-NMR:δ(ppm, CD₃OD) 0.70 (3H, s), 0.81 (3H, s), 1.03 (3H, d, *J* = 6.9 Hz), 1.03 (3H, d, *J* = 6.9 Hz), 1.09 (1H, m), 1.25 (1H, m), 1.26 (3H, s), 1.29 (1H, m), 1.36 (1H, m), 1.37 (1H, m), 1.47 (1H, m), 1.51 (1H, m), 1.54 (1H, m), 1.58 (1H, m), 1.61 (1H, m), 1.62 (1H, m), 1.63 (1H, m), 1.65 (1H, m), 1.66 (1H, m), 1.74 (1H, m), 1.74 (1H, m), 1.76 (2H, m), 1.78 (1H, m), 1.83 (1H, m), 1.84 (1H, m), 2.05 (1H, m), 2.11 (1H, m), 2.11 (1H, m), 2.25 (1H, m), 3.50 (1H, m), 4.67 (1H, d, *J* = 1.4 Hz), 4.73 (1H, s), 5.18 (1H, m).
¹³C-NMR:δ(ppm, CD₃OD 13.43, 14.10, 22.41, 22.41, 22.57, 23.30, 23.63, 25.96, 29.97, 30.83, 32.12, 35.24, 35.34, 38.35, 38.70, 41.28, 41.63, 43.64, 44.65, 50.83, 56.58, 59.69, 71.58, 75.90, 106.76, 118.96, 140.51, 157.76.

### Physicochemical data of compound D

FAB mass spectrum (*m*/*z*): 395 [M-H₂O+H]⁺.
High resolution FAB mass spectrum (*m*/*z*):
Found value 395.3316
Theoretical value 395.3314 (as C₂₈H₄₃O)
¹H-NMR:δ(ppm, CD₃OD 0.76 (3H, s), 0.85 (3H, s), 1.03 (3H, d, *J* = 6.9 Hz), 1.03 (3H, d, *J* = 6.9 Hz), 1.09 (1H, m), 1.24 (1H, m), 1.25 (1H, m), 1.38 (1H, m), 1.41 (1H, m), 1.48 (1H, m), 1.62 (1H, m), 1.67 (1H, m), 1.70 (1H, m), 1.75 (1H, m), 1.77 (1H, m), 1.80 (2H, m), 1.81 (1H, m), 1.83 (1H, m), 1.85 (1H, m), 2.12 (1H, m), 2.14 (1H, m), 2.14 (1H, m), 2.21 (1H, m), 2.22 (1H, m), 2.25 (1H, m), 2.28 (1H, m), 3.51 (1H, m), 4.48 (1H, m), 4.68 (1H, d, *J* = 1.2 Hz), 4.75 (1H, s), 4.88 (1H, s), 4.92 (1H, d, *J* = 0.7 Hz), 5.86 (1H, m).
¹³C-NMR:δ(ppm, CD₃OD) 13.28, 16.73, 22.30, 22.35, 22.53, 31.00, 32.20, 34.73, 35.08, 35.44, 37.73, 38.28, 38.75, 40.86, 41.46, 41.61, 44.85, 51.44, 56.90, 59.87, 71.55, 72.15, 107.19, 110.75, 120.48, 137.17, 150.20, 157.03.

### Physicochemical data of compound E

FAB mass spectrum (*m*/*z*): 415 [M+H]⁺, 413 [M-H]⁻.
¹H-NMR:δ(ppm, CD₃OD) 0.72 (3H, s), 0.94 (3H, s), 0.99 (3H, d, *J* = 6.5 Hz), 1.02 (3H, d, *J* = 6.8 Hz), 1.03 (3H, d, *J* = 6.8 Hz), 1.17 (1H, dddd, *J =* 13.5, 10.7, 8.6, 4.8 Hz), 1.31 (1H, t, *J* = 9.5 Hz), 1.41 (1H, m), 1.43 (1H, m), 1.43 (1H, m), 1.46 (1H, m), 1.46 (1H, m), 1.46 (1H, m), 1.48 (1H, m), 1.59 (1H, dddd, *J =* 13.5, 10.9, 5.9, 2.7 Hz), 1.71 (1H, m), 1.73 (1H, m), 1.84 (1H, m), 1.93 (1H, ddd, *J* = 14.8, 10.2, 5.9 Hz), 2.03 (1H, m), 2.09 (1H, m), 2.13 (1H, m), 2.19 (1H, m), 2.21 (1H, m), 2.24 (1H, m), 2.30 (1H, m), 2.41 (1H, m), 2.50 (1H, dd, *J* = 12.8, 3.4 Hz), 3.56 (1H, m), 4.66 (1H, d, *J* = 1.3 Hz), 4.72 (1H, s).
¹³C-NMR:δ(ppm, CD₃OD) 11.48, 17.11, 19.13, 22.27, 22.27, 22.44, 22.81, 30.00, 30.88, 31.39, 32.07, 33.92, 34.93, 35.16, 35.90, 36.98, 37.22, 45.55, 47.13, 50.10, 55.52, 70.68, 106.96, 119.65, 146.22, 157.67, 173.11.

### Physicochemical data of compound F

FAB mass spectrum (*m*/*z*): 413 [M+H]⁺, 395 [M-H₂O+H]⁺.
High resolution FAB mass spectrum (*mlz*)*:*
Found value 413.3389
Theoretical value 413.3419 (As C₂₈H₄₅O₂)
¹H-NMR:δ(ppm, CD₃OD) 0.63 (3H, s), 1.00 (3H, d, *J* = 6.4 Hz), 1.02 (3H, d, *J =* 6.8 Hz), 1.03 (3H, d, *J* = 6.8 Hz), 1.15 (1H, m), 1.18 (1H, m), 1.20 (3H, s), 1.33 (1H, m), 1.33 (1H, m), 1.40 (1H, m), 1.43 (1H, m), 1.43 (1H, m), 1.43 (1H, m), 1.52 (1H, m), 1.59 (1H, m), 1.63 (1H, m), 1.88 (1H, m), 1.89 (1H, m), 1.93 (1H, m), 1.95 (1H, m), 1.95 (1H, m), 2.09 (1H, dd, *J* = 16.2, 3.7 Hz), 2.10 (1H, m), 2.12 (1H, m), 2.14 (1H, m), 2.23 (1H, m), 2.38 (1H, m), 2.42 (1H, m), 2.42 (1H, m), 2.51 (1H, m), 3.56 (1H, m), 4.65 (1H, d, *J* = 1.5 Hz), 4.72 (1H, s).
¹³C-NMR:δ(ppm, CD₃OD) 11.75, 17.49, 19.28, 22.28, 22.44, 26.00, 26.51, 30.23, 32.00, 32.10, 34.92, 35.49, 35.92, 36.94, 37.24, 38.21, 39.65, 42.53, 43.46, 43.73, 49.57, 54.74, 70.58, 106.93, 134.04, 157.74, 168.71, 201.45.

### Physicochemical data of compound G

¹H-NMR:δ(ppm, CDCl₃) 4.72 (1H, s), 4.66 (1H, s), 2.50 (1H, m), 2.46 (1H, m), 2.43 (1H, m), 2.41 (1H, m), 2.39 (1H, m), 2.37 (1H, m), 2.23 (2H, m), 2.21 (1H, m), 2.17 (1H, m), 2.15 (1H, m), 2.11 (1H, m), 2.09 (1H, m), 1.98 (1H, m), 1.89 (1H, m), 1.74 (1H, ddd, *J* = 13.1, 13.1, 5.8 Hz), 1.58 (1H, m), 1.46 (1H, m), 1.45 (1H, m), 1.37 (3H, s), 1.35 (2H, m), 1.18 (1H, m), 1.17 (1H, m), 1.03 (3H, d, *J* = 6.8 Hz), 1.02 (3H, d, *J =* 6.8 Hz), 0.98 (3H, d, *J* = 6.6 Hz), 0.63 (3H, s).
¹³C-NMR:δ(ppm, CDCl₃) 208.9 (s), 197.5 (s), 163.0 (s), 156.7 (s), 134.0 (s), 106.1 (t), 53.5 (d), 48.3 (d), 43.7 (t), 42.52 (s), 42.46 (d), 42.3 (t), 38.2 (s), 37.9 (t), 36.1 (d), 35.8 (t), 35.7 (t), 34.6 (t), 33.8 (d), 31.1 (t), 29.2 (t), 25.7 (t), 24.8 (t), 22.0 (q), 21.9 (q), 18.9 (q), 16.4 (q), 11. 5 (q),

### Physicochemical data of compound H

FAB mass spectrum (*mlz*)*:* 481 [M+Na]⁺, 459 [M+H]⁺, 341 [M-H₂O+H]⁺, 457 [M-H]⁻.
High resolution FAB mass spectrum (*m*/*z*):
Found value 459.3080
Theoretical value 459.3110 (as C₂₈R₄₃O₅)
¹H-NMR:δ(ppm, CD₃OD) 0.95 (3H, s), 1.02 (1H, m), 1.04 (3H, d, *J* = 6.8 Hz), 1.04 (3H, d, *J* = 6.8 Hz), 1.15 (1H, m), 1.33 (1H, m), 1.38 (1H, m), 1.47 (3H, s), 1.53 (1H, dd, *J* = 13.0, 3.9 Hz), 1.59 (1H, m), 1.64 (1H, dd, *J* = 15.4, 12.9 Hz), 1.72 (1H, m), 1.74 (1H, m), 1.79 (1H, m), 1.84 (1H, m), 1.85 (1H, m), 1.89 (1H, m), 1.89 (2H, m), 1.90 (1H, m), 2.01 (1H, m), 2.08 (1H, m), 2.18 (1H, d, *J* = 2.5 Hz), 2.28 (1H, m), 2.36 (1H, m), 2.40 (1H, m), 2.44 (1H, dd, *J* =7.5, 6.3 Hz), 3.36 (1H, d, *J* =5.8 Hz), 3.47 (1H, m), 4.12 (1H, dd, *J* = 4.4, 2.2 Hz), 4.74 (1H, d, *J* = 1.2 Hz), 4.79 (1H, s).
¹³C-NMR:δ(ppm, CD₃OD) 12.98, 20.95, 22.29, 22.29, 27.69, 29.68, 29.83, 31.49, 35.14, 35.53, 35.93, 36.87, 38.28, 38.57, 39.01, 41.21, 50.69, 53.52, 54.92, 54.98, 55.50, 63.93, 71.42, 71.47, 85.84, 107.33, 156.54, 180.61.

### Physicochemical data of compound I

ESI-MS *m*/*z* 435 [M+H]⁺
¹H NMR:δ(ppm, CDCl₃, 400 MHz) 9.72 (1H, br.s), 7.52 (1H, d, *J* = 8.5Hz), 6.66 (1H, d, *J* = 8.5Hz), 5.62 (1H, br.s), 3.19 (1H, d, *J* = 10.2Hz), 3.14 (1H, ddd, *J* = 8.4, 8.4, 3.5Hz), 2.93 (1H, d, *J* = 17.2Hz), 2.78 (1H, d, *J* = 17.4Hz), 2.77 (2H, s), 2.69 (1H, d, *J* = 10.2Hz), 2.61 (1H, ddd, *J* = 12.2, 9.1, 4.7Hz), 2.39 (1H, dd, *J* = 17.2, 8.6Hz), 2.17 (1H, m), 2.16 (1H, m), 1.98 (1H, dd, *J* = 11.8, 2.6Hz), 1.95 (2H, m), 1.50 (3H, s), 1.50 (3H, s), 1.47 (1H, ddd, *J* = 12.2, 11.0, 7.0Hz), 1.31 (3H, s), 1.23 (3H, s).
¹³ C NMR:δ(ppm, CDCl₃, 100 MHz) 194.15 (s), 173.69 (s), 157.60(s), 141.45 (s), 134.29 (s), 126.85 (d), 121.38 (s), 109.83 (d), 105.31 (s), 103.19 (s), 79.61 (s), 65.34 (s), 62.46 (t), 55.95 (s), 53.50 (t), 48.81 (t), 46.18 (d), 34.31 (s), 31.96 (t), 29.24 (t), 27.91 (q), 27.33 (t), 26.70 (q), 26.64 (q), 24.73(q), 22,89 (t).

### Physicochemical data of compound J

FAB mass spectrum (*m*/*z*): 413 [M-H₂O+H]⁺, 395 [M-2H₂O+H]⁺.
High resolution FAB mass spectrum (*m*/*z*):
Found value 413.3422
Theoretical value 413.3419 (as C₂₈H₄₅O₂)
¹H-NMR:δ(ppm, CD₃OD) 0.85 (3H, s), 1.01 (3H, s), 1.03 (3H, d, *J* = 6.8 Hz), 1.04 (3H, d, *J* = 6.8 Hz), 1.09 (1H, m), 1.24 (1H, m), 1.24 (1H, m), 1.28 (3H, s), 1.38 (1H, m), 1.40 (1H, m), 1.51 (1H, m), 1.56 (1H, m), 1.56 (1H, m), 1.57 (1H, m), 1.62 (1H, m), 1.66 (1H, m), 1.67 (1H, m), 1.68 (1H, m), 1.75 (1H, m), 1.79 (2H, m), 1.80 (1H, m), 1.83 (1H, m), 2.05 (1H, m), 2.06 (1H, m), 2.10 (1H, m), 2.25 (1H, m), 2.25 (1H, m), 3.50 (1H, m), 4.43 (1H, m), 4.67 (1H, d, *J* = 1.2 Hz), 4.73 (1H, s), 5.84 (1H, m).
¹³C-NMR:δ(ppm, CD₃OD) 13.26, 17.36, 22.32, 22.41, 22.41, 25.76, 29.80, 31,04, 32.19, 35.28, 35.38, 37.53, 38.26, 38.75, 41.44, 42.88, 43.47, 44.27, 51.26, 59.79, 60.50, 71.56, 71.56, 75.68, 106.76, 120.74, 137.10, 157.76.

### Physicochemical data of compound K

ESI mass spectrum (*mlz*): 393.4 [M-H₂O+H]⁺.
¹H-NMR:δ(ppm, CDCl₃) 0.82 (3H, s), 1.04 (6H, d, *J* = 6.8 Hz), 1.06 (3H, s), 1.29 (1H, ddd, *J* = 12.7, 12.7, 4.2 Hz), 1.47 (1H, ddd, *J* = 14.4, 14.4, 4.4 Hz), 1.53 (1H, m), 1.68 (1H, m), 1.93□1.82 (7H, m), 2.37-2.12 (11H, m), 4.53 (1H, m), 4.68 (1H, s), 4.76(1H, s), 4.91 (1H, s), 4.96 (1H, s), 5.83 (1H, m).
¹³C-NMR:δ(ppm, CDCl₃) 12.3, 16.5, 21.5, 21.9, 22.0, 30.2, 33.4, 34.1, 34.5, 36.4, 38.2, 38.8, 39.5, 40.1, 42.8, 43.6, 44.2, 49.5, 58.8, 71.6, 106.5, 110.4, 118.9, 136.3, 148.4, 155.8, 211.6.

### Physicochemical data of compound L

ESI mass spectum (*m*/*z*): 425.4 [M +H]⁺, 407.4 [M-H₂O+H]⁺.
¹H-NMR:δ(ppm, CDCl₃) 0.92 (3H, s), 1.03 (6H, d, *J*=6.8 Hz), 1.30 (1H, m), 1.38 (3H, s), 1.52 (1H, m), 2.12-1.72 (13H; m), 2.34-2.21 (4H, m), 2.47 (1H, ddd, *J* = 14.2, 4.6, 2.2 Hz), 3.64 (1H, m), 4.37 (1H, s), 4.72 (1H, s), 4.76 (1H, s), 5.33 (1H, s), 5.59 (1H, dd, *J* = 5.6, 2.4 Hz), 5.76 (1H, m).
¹³C-NMR:δ(ppm, CDCl₃) 16.3, 21.9, 21.9, 22.2, 26.7, 27.5, 29.5, 32.0, 34.0, 34.7, 37.2, 38.5, 39.8, 40.9, 45.9, 48.4, 55.2, 58.8, 70.7, 74.4, 86.0, 105.9, 106.5, 118.3, 119.3, 133.6, 140.9,155.7.

### Physicochemical data of compound M

¹H-NMR:δ(ppm, CDCl₃, 400 MHz) 7.97 (1H, br s), 7.33 (1H, d, *J* = 7.8 Hz), 7.25 (1H, d, *J* = 7.8 Hz), 7.09 (1H, t, *J* = 7.8 Hz), 6.77 (1H, d, *J* = 16.4 Hz), 6.26 (1H, d, *J* = 16.4 Hz), 5.69 (1H, br s), 3.28 (3H, s), 3.21 (1H, d, *J* = 10.0 Hz), 3.15 (1H, m), 2.96 (1H, d, *J* = 17.3 Hz), 2.81 (1H, d, *J* = 17.3 Hz), 2.69 (1H, d, *J* = 10.0 Hz), 2.62 (1H, m), 2.40 (1H, m), 2.19 (2H, m), 1.98 (1H, m), 1.95 (2H, m), 1.50 (1H, m), 1.440 (3H, s), 1.436 (3H, s), 1.33 (3H, s), 1.25 (3H, s).
¹³C-NMR:δ(ppm, CDCl₃, 100 MHz) 173.7 (s), 141.7 (s), 136.6 (d), 134.2 (s), 127.7 (s), 124.6 (d), 120.6 (s), 120.0 (d), 119.9 (d), 117.3 (d), 103.8 (s), 75.4 (s), 65.4 (s), 62.4 (t), 55.9 (s), 53.5 (t), 50.6 (q), 46.2 (d), 34.3 (s), 32.0 (t), 29.4 (t), 27.9 (q), 27.3 (t), 26.2 (q), 26.1 (q), 24.9 (q), 22.9 (t).

### Physicochemical data of compound N

¹H-NMR:δ(ppm, CDCl₃, 500 MHz) 10.31 (1H, br s), 7.77 (1H, d, *J* = 7.6 Hz), 7.64 (1H, d, *J* = 7.6 Hz), 7.14 (1H, t, *J* = 7.6 Hz), 6.93 (1H, t, *J* = 1.2 Hz), 5.68 (1H, br s), 3.22 (1H, d, J = 10.1 Hz), 3.15 (1H, m), 2.99 (1H, d, *J* = 17.2 Hz), 2.84 (1H, d, *J* = 17.2 Hz), 2.71 (1H, d, *J* = 10.1 Hz), 2.62 (1H, m), 2.38 (1H, m), 2.25 (3H, d, *J* = 1.2 Hz), 2.21 (1H, m), 2.15 (1H, m), 2.05 (3H, d, *J* = 1.2 Hz), 2.00 (1H, m), 1.95 (2H, m), 1.46 (1H, m), 1.36 (3H, s), 1.26 (3H, s). ¹³C-NMR:δ(ppm, CDCl₃, 125 MHz) 193.3 (s), 173.6 (s), 155.3 (s), 143.3 (s), 136.1 (s), 128.6 (s), 123.8 (d), 123.6 (d), 121.2 (s), 121.0 (d), 118.5 (d), 102,8 (s), 65.4 (s), 62.5 (t), 56.0 (s), 53.5 (t), 46.2 (d), 34.4 (s), 32.0 (t), 29.3 (t), 28.0 (q), 27.9 (q), 27.3 (t), 24.8 (q), 22.9 (t), 21.1 (q).

### Physicochemical data of compound O

¹H-NMR:δ(ppm,CDCl₃, 400 MHz) 7.83 (1H, br s), 7.29 (1H, d, *J* = 7.3 Hz), 7.05 (1H, t, *J* = 7.3 Hz), 6.98 (1H, d, *J* = 7.3 Hz), 5.67 (1H, br s), 5.42 (1H, m), 3.57 (2H, m), 3.21 (1H, d, *J* = 10.3 Hz), 3,15 (1H, m), 2.96 (1H, d, *J* = 17.1 Hz), 2.81 (1H, d, *J* = 17.1 Hz), 2.69 (1H, d, *J* = 10.3 Hz), 2.62 (1H, m), 2.40 (1H, m), 2.19 (2H, m), 1.97 (3H, m), 1.86 (3H, s), 1.79 (3H, d, *J* = 1.2 Hz), 1.48 (1H, m), 1.27 (3H, s), 1.21 (3H, s).
¹³C-NMR:δ(ppm, CDCl₃, 100 MHz) 174.0 (s), 141.1 (s), 135.6 (s), 133.2 (s), 127.0 (s), 123.6 (s), 122.5 (d), 121.7 (d), 119.9 (d), 115.9 (d), 103.5 (s), 65.5 (s), 62.4 (t), 56.0 (s), 53.5 (t), 46.1 (d), 34.3 (s), 32.0 (t), 30.9 (t), 29.5 (t), 27.9 (q), 27.3 (t), 25.7 (q), 25.0 (q), 22.9 (t), 18.0 (q).

### Physicochemical data of compound P

ESI-MS *m*/*z* 450.2 [M+H]⁺, 448.1 [M-H]'.
¹H-NMR:δ(ppm, CDCl₃, 400 MHz) 9.20 (1H, br s), 7.54 (1H, br s), 7.42 (1H, d, *J* = 8.3 Hz), 6.54 (1H, d, *J* = 8.3 Hz), 3.62 (1H, d, *J* = 8.8 Hz), 3.32 (1H, t, *J* = 8.8 Hz), 3.14 (1H, m), 2.74 (1H, d, *J* = 16.6 Hz), 2.68 (1H, d, *J* = 16.6 Hz), 2.64 (1H, m), 2.48 (1H, d, *J* = 8.8 Hz), 2.32 (1H, d, *J* = 15.1 Hz), 2.21 (1H, m), 2.07 (1H, d, *J* = 15.1 Hz), 1.97 (3H, m), 1.67 (1H, dd, *J* = 12.7, 8.8 Hz), 1.50 (1H, m), 1.49 (3H, s), 1..47 (3H, s), 0.98 (3H, s), 0.78 (3H, s).
¹³C-NMR:δ(ppm, CDCl₃, 100 MHz) 193.7 (s), 183.3 (s), 174.6 (s), 159.3 (s), 142.9 (s), 133.0 (d), 121.5 (s), 109.6 (d), 104.9 (s), 79.4 (s), 66.9 (s), 63.3 (s), 63.0 (t), 61.7 (s), 53.6 (t), 48.8 (t), 48.6 (d), 47.1 (s), 40.2 (t), 32.0 (t), 26.8 (q), 26.74 (q), 26.70 (t), 23.9 (t), 22.9 (q), 21.1(q).

### Physicochemical data of compound Q

ESI-MS *m*/*z* 450.2 [M+H]⁺.
¹H-NMR:δ(ppm, CDCl₃, 400 MHz) 10.54 (1H, s), 7.67 (1H, br s), 7.43 (1H, d, *J* = 8.3 Hz), 6.49 (1H, d, *J* = 8.3 Hz), 3.60 (1H, d, *J* = 8.8 Hz), 3.16 (1H, t, *J* = 7.8 HZ), 3.11 (1H, m), 2.86 (1H, d, *J* = 16.8 Hz), 2.63 (1H, m), 2.55 (1H, d, *J* = 16.8 Hz), 2.48 (1H, d, *J* = 8.8 Hz), 2.29 (1H, d, *J* = 15.4 Hz), 2.20 (1H, m), 2.08 (1H, d, *J* = 15.4 Hz), 1.96 (3H, m), 1.76 (3H, s), 1.65 (1H, dd, *J* = 12.7, 7.8 Hz), 1.50 (1H, m), 1.44 (3H, s), 0,94 (3H, s), 0.81 (3H, s).
¹³C-NMR:δ(ppm, CDCl₃, 100 MHz) 196.7 (s), 182.3 (s), 174.8 (s), 159.5 (s), 147.0 (s), 134.2 (d), 118.3 (s), 108.3 (d), 103.9 (s), 66.8 (s), 64.2 (s), 63.2 (s), 62.9 (t), 56.9 (s), 53.5 (t), 51.5 (t), 48.7 (d), 47.2 (s), 40.2 (t), 32.1 (t), 26.73 (t), 26.68 (q), 23.92 (t), 23.88 (q), 23.1 (q), 21.0 (q).

### Physicochemical data of compound R

ESI-MS *m*/*z* 442.2 [M-H₂O+H]⁺, 458.2 [M-H]
¹H-NMR:δ(ppm, CDCl₃, 400 MHz) 8.33 (1H, dd, *J* = 8.1, 1.5 Hz), 7.79 (1H, ddd, *J* = 8.3, 7.1, 1.5 Hz), 7.71 (1H, d, *J* = 8.3 Hz), 7.54 (1H, d, *J* = 7.8 Hz), 7.53 (1H, ddd, *J* = 8.1, 7.1, 1.5 Hz), 7.33 (1H, ddd, *J =* 7.8, 7.6, 1.2 Hz), 7.30 (1H, d, *J* = 7.3 Hz), 7.10 (1H, ddd, *J* = 7.6, 7.6, 1.2 Hz), 6.06 (1H, br s), 5.88 (1H, m), 5.28 (1H, s), 4.91 (1H, br s), 4.80 (1H, q, *J* = 6.6 Hz), 2.76 (1H, dd, *J* = 14.7,3.7 Hz), 2.45 (1H, dd, *J* = 14.7,9.0 Hz), 1.81 (3H, d, *J* = 6.6 Hz), 1.53 (3H, s), 1.42 (3H, s).
¹³C-NMR:δ(ppm, CDCl₃, 125 MHz) 175.2 (s), 169.6 (s), 160.4 (s), 150.7 (s), 146.9 (s), 138.0 (s), 137.5 (s), 134.8 (d), 130.2 (d), 127.6 (d), 127.2 (d), 125.2 (d), 124.0 (d), 120.5 (s), 115.8 (d), 78.4 (d), 74.1 (s), 64.8 (s), 52.0 (d), 49.5 (d), 39.5 (t), 26.4 (q), 25.4 (q), 17.4 (q).

### Physicochemical data of compound S

ESI-MS *m*/*z* 458.2 [M+H]⁺, 456.2 [M-H]'.
¹H-NMR:δ(ppm, CDCl₃, 500 MHz) 8.29 (1H, dd, *J* = 7.9. 1.1 Hz), 7.79 (1H, ddd, *J* = 8.3, 7.1, 1.2 Hz), 7.73 (1H, dd, *J* = 8.3, 1.2 Hz), 7.50 (1H, ddd, *J* = 7.9, 7.1, 1.2 Hz), 7.49 (1H, d, *J* = 7.9 Hz), 7.34 (1H, d, *J* = 7.6 Hz), 7.28 (1H, ddd, *J* = 7.9, 7.7, 1.2 Hz), 7.20 (1H, br s), 7.06 (1H, ddd, *J* = 7.7, 7.6, 1.0 Hz), 5.74 (1H, dd, *J* = 8.0, 3.3 Hz), 5.21 (1H, s), 2.95 (1H, dd, *J* = 15.2, 3.3 Hz), 2.90 (1H, dd, *J* = 15.2, 8.0 Hz), 2.08 (3H, s), 1.39 (3H, s), 136 (3H, s).
¹³C-NMR:δ(ppm, CDCl₃, 100 MHz) 175.4 (s), 170.6 (s), 160.9 (s), 150.2 (s), 146.7 (s), 137.8 (s), 137.5 (s), 135.0 (d), 130.2 (d), 128.0 (d), 127.8 (d), 127.0 (d), 125.2 (d), 124.3 (d), 120.7 (s), 115.6 (d), 81.2 (s), 79.1 (d), 74.8 (s), 65.1 (s), 52.8 (d), 42.1 (t), 27.3 (q), 26.0 (q), 25.0 (q).

### Physicochemical data of compound T

ESI-MS *m*/*z* 444.2 [M+H]⁺, 442.1 [M-H]⁻.
¹H-NMR:δ(ppm, CDCl₃, 400 MHz) 8.20 (1H, dd, *J* = 8.1, 1.2 Hz), 7.77 (1H, ddd, *J* = 7.1, 7.1, 1.5 Hz), 7.72 (1H, br s), 7.71 (1H, d, *J* = 7.1 Hz), 7.5 0 (1H, ddd, *J* = 8.1, 7.1, 1.2 Hz), 7.36 (1H, d, *J* = 7.6 Hz), 7.30 (1H, d, *J* = 7.3 Hz), 7.24 (1H, ddd, *J* = 7.6, 7.6, 1.2 Hz), 7.08 (1H, ddd, *J* = 7.6, 7.3, 1.0 Hz), 5.61 (1H, dd, *J* = 4.9, 2.2 Hz), 4.79 (1H, d, *J* = 1.2 Hz), 4.49 (1H, br s), 4.40 (1H, q, *J* = 6.8 Hz), 3.34 (1H, dd, *J* = 15.4, 4.9 Hz), 2.56 (1H, dd, *J* = 15.4, 2.2 Hz), 2.11 (3H, s), 1.59 (3H, d *J* = 6.8 Hz).
¹³CMR:δ(ppm, CDCl₃, 100 MHz) 170.2 (s), 169.0(s), 160.1 (s), 149.7 (s), 146.8 (s), 138.7 (s), 136.3 (s),135.1 (d), 130.2 (d), 127.9 (d), 127.8 (d), 126.9 (d), 125.8 (d), 123.4 (d), 120.3 (s), 116.4 (d), 82.6 (d), 77.3 (s), 71.6 (s), 63.2 (d), 54.1 (d), 37.4 (t), 24.8 (q), 17.3 (q).

### EXAMPLE 3

### Production of compounds U, V, W and X

As the flask seed medium, a medium comprising glucose (20 g/L), mashed potatoes (30 g/L) and dry yeast extract (5 g/L) (pH 6.5) was used, and as the fermentation medium, a medium comprising sucrose (30 g/L), soluble starch (20 g/L), corn steep liquor (CSL) (30 g/L), malt extract (20 g/L) and calcium carbonate (5 g/L) (pH 5.0) was used. About 2 mL of spore suspension (3x10⁸ spores/mL) of *Penicillium* sp. CND1007 was inoculated into the first seed medium (50 mL) in a 250 mL capacity conical flask, followed by shaking culturing (the number of revolutions 220 rpm) at 25°C for 48 hours. Next, a full amount of the first seed culture liquid (about 50 mL) was inoculated into the second seed liquid (400 mL) in a 2 L capacity conical flasks, followed by shaking culturing (the number of revolutions 220 rpm) at 25°C for 24 hours in the same manner. Subsequently, a full amount of the second seed culture liquid (390 mL) was inoculated into the fermentation medium (about 15 L) which had been put into a 30 L capacity jar fermenter, followed by aeration agitation culturing (the number of agitations: 100 rpm; aeration flow rate: 10 L/min) 25°C for 24 hours. Furthermore, the third seed culture liquid (about 3.9 L) was inoculated into the main fermentation medium (130 L) which had been put into a 200 L capacity jar fermenter, followed by aeration agitation culturing (the number of revolutions: 150 rpm; aeration flow rate: 75 L/min) at 25°C for 144 hours.

A filter aid (Radiolite # 600, manufactured by Showa Chemical Industry) was added at a ratio of 10% by weight to the thus obtained fermentation culture liquid (about 120 L), and then the culture filtrate and mycelia were separated by suction filtration. The separated mycelia were mixed with 30 L of methanol, followed by extraction twice at room temperature. The extract (60 L) was diluted by adding water (120 L) and applied to a column filled with 5 L of Diaion HP 20 (manufactured by Mitsubishi Chemical Corp.) to adsorb the compounds of interest. After washing with 33% methanol (2 L x4) and 66% methanol (2 Lx4), the compounds of interest were eluted with 100% methanol (2 Lx4), 33% acetone/methanol (2 Lx 4) and 100% acetone (2 L). The eluate (about 18 L) was concentrated under a reduced pressure to about 5 L and then diluted with water (about 5 L), followed by extraction twice with chloroform (10 L). The extract was concentrated under reduced pressure, and the thus obtained residue (138 g) was applied to a column filled with 2 L of silica gel and eluted stepwise using n-hexane (2 Lx2), 25% ethyl acetate/n-hexane (2 Lx2), 50% ethyl acetate/n-hexane (2 Lx2), 75% ethyl acetate/n-hexane (2 Lx 2), ethyl acetate (2 Lx 2), 25% methanol/ethyl acetate (2 Lx 2) and 50% methanol/ethyl acetate (2 Lx 2). Residue (16.6 g) was obtained by concentrating the 50% and 75% ethyl acetate/n-hexane eluates under reduced pressure. The residue was fractionated by silica gel column chromatography (500 mL capacity; 0 to 100% ethyl acetate/n-hexane) to obtain a fraction (4.88 g) containing the compounds U, V, W and X. The fraction (4.88 g) containing the compounds U, V, W and X was separated and purified by fractional high performance liquid chromatography [column SunFire^{™} Prep C18 OBD 10 µm (manufactured by Waters), φ 19x250 mm, column temperature: 40°C, flow rate: 10 mL/min, stepwise elution with 85 to 100% methanol aqueous solution or acetonitrile aqueous solution], to obtain each of the compound U (1.7 mg), compound V (2.9 mg), compound W (10.7 mg) and compound X (3.4 mg).

### EXAMPLE 4

### Production of compounds E, Y, Z, AA and BB

A fermentation culture liquid obtained in accordance with the method described in Example 3 was separated into culture filtrate and cell bodies by suction filtration. The separated cell bodies were mixed with 30 L of methanol, followed by extraction twice at room temperature. The extract (60 L) was diluted by adding water (120 L) and applied to a column filled with 5 L of Diaion HP 20 (manufactured by Mitsubishi Chemical Corp.), to adsorb the compounds of interest. After washing with 33% methanol (2 Lx4) and 66% methanol (2 Lx4), the compounds of interest were eluted with 100% methanol (2 Lx4), 33% acetone/methanol (2 Lx4) and 100% acetone (2 L×3). The eluate (about 22 L) was concentrated under reduced pressure to about 8 L and then diluted by adding water (about 7 L), followed by extraction three times with chloroform (10 L). The extract was concentrated under reduced pressure, and the thus obtained residue (81.3 g) was applied to a column filled with 2.5 L of silica gel and eluted stepwise using n-hexane (2 Lx3), 25% ethyl acetate/n-hexane (2 Lx2), 50% ethyl acetate/n-hexane (2 Lx2), 75% ethyl acetate/n-hexane (2 Lx2), ethyl acetate (2 Lx2), 25% methanol/ethyl acetate (2 Lx2) and 50% methanol/ethyl acetate (2 Lx2). Residue (2.4 g) obtained by concentrating the 50% ethyl acetate/n-hexane eluate under reduced pressure was mixed with methanol. A precipitated solid was collected by filtration to obtain compound E (about 400 mg). Subsequently, the filtrate was separated and purified by fractional high performance liquid chromatography [column SunFire^{™} Prep C18 OBD 10 µm (manufactured by Waters), φ 19x250 mm, column temperature: 40°C, flow rate: 10 mL/min, stepwise elution with 85 to 100% acetonitrile aqueous solution], to obtain each of compound Y (38.8 mg), compound Z (12.1 mg), compound AA (23.4 mg) and compound BB (9.4 mg).

Physicochemical data of the compounds U to BB obtained in the above-mentioned Examples 3 and 4 are as follows.

### Physicochemical data of compound U

¹H NMRδ(ppm, CDCl₃, 500 MHz) 4.73 (1H, s), 4.66 (1H, d, *J* = 1.4 Hz), 3.63 (1H, m), 2.49 (1H, m), 2.40 (1H, m), 2.26 (1H, m), 2.23 (1H, m), 2.21 (1H, m), 2.20 (1H, m), 2.09 (1H, m), 2.00 (1H, ddd, *J* = 13.1, 3.3, 3.3 Hz), 1.95 (1H, m), 1.91 (1H, m), 1.90 (1H, m), 1.74 (1H, m), 1.72 (1H, m), 1.63 (1H, m), 1.58 (1H, m), 1.51 (1H, m), 1.49 (1H, m), 1.47 (1H, m), 1.44 (1H, m), 1.34 (1H, ddd, *J* = 13.1, 2.9, 2.9 Hz), 1,28 (1H, m), 1.21 (1H, m), 1.20 (1H, m), 1.03 (3H, d, *J* = 6.8 Hz), 1.02 (3H, d, *J* = 6.9 Hz), 0.97 (3H, d, *J* = 6.7 Hz), 0.93 (3H, s), 0.85 (3H, s).
¹³C NMRδ(ppm, CDCl₃, 125 MHz) 169.4 (s), 156.6 (s), 140.3 (s), 129.9 (s), 106.2 (t), 70.6 (d), 56.7 (d), 49.8 (d), 47.0 (d), 44.3 (s), 36.4 (t), 34.8 (d), 34.42 (t), 34.40 (t), 33.9 (2C, d & s), 31.4 (t), 30.9 (t), 30.3 (t), 27.8 (t), 22.6 (t), 22.0 (q), 21.9 (q), 20.7 (t), 19.0 (q), 18.8 (q), 13.2 (q).

### Physicochemical data of compound V

¹H NMRδ(ppm, CDCl₃, 500 MHz) 5.76 (1H, m), 4.74 (1H, s), 4.67 (1H, d, *J* = 1.4 Hz), 3.64 (1H, m), 2.48 (1H, m), 2.43 (1H, dd, *J* = 12.7, 3.4 Hz), 2.35 (1H, m), 2.30 (2H, m), 2.24 (1H, m), 2.16 (1H, m), 2.12 (1H, m), 2.08 (1H, m), 1.94 (1H, m), 1.93 (1H, m), 1.76 (1H, m), 1.67 (1H, m), 1.63 (1H, m), 1.60 (1H, m), 1.59 (1H, m), 1.49 (1H, m), 1.47 (1H, m), 1.45 (1H, m), 1.23 (1H, m), 1.04 (3H, d, *J* = 6.7 Hz), 1.031 (3H, d, *J* = 6.8 Hz), 1.030 (3H, s), 0.98 (3H, d, *J* = 6.2 Hz), 0.87 (3H, s).
¹³C NMRδ(ppm, CDCl₃, 125 MHz) 170.1 (s), 156.6 (s), 141.6 (s), 140.7 (s), 121.9 (s), 121.1 (d), 106.2 (t), 70.2 (d), 57.1 (d), 46.9 (s), 46.1 (d), 36.4 (t), 35.8 (t), 34.7 (s), 34.6 (t), 33.9 (2C, d), 33.4 (t), 30.9 (t), 30.8 (t), 30.1 (t), 22.0 (q), 21.9 (q), 21.2 (t), 18.9 (q), 17.5 (q), 15.6 (q).

### Physicochemical data of compound W

¹H NMRδ(ppm, CDCl₃ 500 MHz) 4.71 (1H, s), 4.65 (1H, d, *J* = 1.4 Hz), 3.61 (1H, m), 2.36 (1H, t, *J* = 12.7 Hz), 2.35 (1H, t, *J* = 11.2 Hz), 2.22 (1H, m), 2.20 (1H, m), 2.09 (1H, m), 2.03 (1H, dd, *J* = 12.7, 3.4 Hz), 1.99 (1H, m), 1.92 (1H, m), 1.88 (1H, m), 1.87 (1H, m), 1.77 (1H, m), 1.62 (1H, m), 1.56 (1H, m), 1.55 (1H, m), 1.53 (1H, m), 1.50 (1H, m), 1.48 (1H, m), 1.44 (1H, m), 1.42 (1H, m), 1.41 (1H, m), 1.27 (1H, m), 1.17 (1H, m), 1.14 (1H, m), 1.11 (1H, m), 1.10(1H,m), 1.08(3H, s), 1.03 (3H, d, *J* = 6.8 Hz),1.02 (1H, m),1.01 (3H, d, *J* = 6.9 Hz), 0.96 (1H, m), 0.94 (3H, d, *J* = 6.6 Hz), 0.66 (3H, s).
¹³C NMRδ(ppm, CDCl₃, 125 MHz) 212.1 (s), 156.8 (s), 106.1 (t), 70.8 (d), 55.3 (d), 54.9 (d), 50.0 (d), 48.9 (d), 46.9 (d), 46.1 (t), 42.6 (s), 38.8 (t), 38.0 (t), 36.1 (t), 36.0 (s), 35.6 (d), 34.7 (t), 33.8 (d), 31.1 (t), 31.0 (t), 28.4 (t), 25.0 (t), 22.0 (q), 21.91 (t), 21.89 (q), 18.8 (q), 12.1 (q), 11.9 (q).

### Physicochemical data of compound X

¹H NMRδ(ppm, CDCl₃, 500 MHz) 5.92 (1H, d, *J* = 15.8 Hz), 5.58 (1H, dd, *J* = 15.8, 8.8 Hz), 4.84 (1H, s), 4.82 (1H, s), 3.61 (1H, m), 2.54 (1H, m), 2.35 (2H, t, *J* = 12.2 Hz), 2.18 (1H, m), 2.12 (1H, m), 2.03 (1H, dd, *J* = 12:2, 3.2 Hz), 1.97 (1H, m), 1.85 (1H, m), 1.77(1H, m), 1.73 (1H, m), 1.63 (1H, m), 1.58 (1H, m), 1.52 (1H, m), 1.51 (1H, m), 1.50 (1H, m), 1.47 (1H, m), 1.46 (1H, m), 1.27 (1H, m), 1.21 (1H, m), 1.12 (1H, m), 1.10 (1H, m), 1.09 (3H, s), 1.08 (6H, d, *J* = 6.4 Hz), 1.06 (3H, d, *J* = 6.5 Hz), 1.02 (1H, m), 0.96 (1H, m), 0.69 (3H, s).
¹³C NMRδ(ppm, CDCl₃, 125 MHz) 211.9 (s), 153.1 (s), 135.9 (d), 129.4 (d), 109.7 (t), 70.8 (d), 55.3 (d), 55.0 (d), 50.0 (d), 49.0 (d), 46.8 (d), 46.1 (t), 42.6 (s), 40.2 (d), 38.7 (t), 38.0 (t), 36.2 (t), 36.0 (s), 31.1 (t), 29.5 (d), 28.5 (t), 25.0 (t), 22.4 (q), 22.1 (q), 21.9 (t), 20.7 (q), 12.4 (q), 11.9 (q).

### Physicochemical data of compound Y

¹H NMRδ(ppm, CDCl₃, 500 MHz) 4.87 (1H, t, *J* = 2.7 Hz), 4.79 (1H, t, *J* = 6.8 Hz), 4.75 (1H, s), 4.68 (1H, d, *J* = 1.2 Hz), 3.67 (1H, m), 2.29 (1H, m), 2.24 (1H, m), 2.18 (1H, dd, *J* = 10.9, 7.7 Hz), 2.06 (1H, m), 2.01 (2H, m), 1.90 (1H, ddd, *J* = 13.3, 13.3, 5.9 Hz), 1.84 (1H, m), 1.83 (1H, m), 1.71 (1H, m), 1.67 (1H, m), 1.63 (1H, m), 1.61 (1H, m), 1.59 (2H, m), 1.53 (1H, m), 1.52 (1H, m), 1.41 (1H, dd, *J* = 13.1, 7.2 Hz), 1.37 (1H, m), 1.32 (3H, s), 1.26 (1H, m), 1.22 (3H, s), 1,19 (1H, m), 1.16 (1H, m), 1.034 (3H, d, *J* = 6.8 Hz), 1.032 (3H, d, *J* = 6.9 Hz), 0.71 (3H, s).
¹³C NMRδ(ppm, CDCl₃, 125 MHz) 156.0 (s), 149.2 (s), 134.8 (s), 106.5 (t), 74.6 (s), 71.0 (d), 69.0 (d), 65.2 (d), 56.3 (d), 43.8 (d), 42.5 (s), 42.3 (t), 37.7 (t), 37.6 (t), 37.2 (s), 37.1 (d), 36.3 (t), 36.2 (t), 34.0 (d), 33.8 (t), 31.4 (t), 28.9 (t), 26.5 (q), 22.0 (q), 21.9 (q), 20.9 (q), 19.2 (t), 11.9 (q).

### Physicochemical data of compound Z

¹H NMRδ(ppm, CDCl₃, 500 MHz) 7.87 (1H, br s), 7.43 (1H, d, *J* = 7.7 Hz), 7.33 (1H, d, *J* = 8.0 Hz), 7.17 (1H, dd, *J* = 8.0, 7.2 Hz), 7.11 (1H, dd, *J* = 7.7, 7.2 Hz), 5.68 (1H, br s), 3.21 (1H, d, *J* = 10.3 Hz), 3.15 (1H, m), 2.96 (1H, d, *J* = 17.2 Hz), 2.81 (1H, d, *J* = 17.2 Hz), 2.70 (1H, d, *J* = 10.3 Hz), 2.62 (1H, m), 2.40 (1H, dd, *J* = 17.2, 8.6 Hz), 2.19 (1H, m), 2.16 (1H, m), 1.97 (1H, dd, *J* = 12.4, 3.2 Hz), 1.94 (2H, m), 1.48 (1H, m), 1.29 (3H, s), 1.22 (3H, s).
¹³C NMRδ(ppm, CDCl₃, 125 MHz) 174.8 (s), 141.5 (s), 136.3 (s), 127.0 (s), 122.0 (d), 119.7 (d), 117.9 (d), 110.8 (d), 103.2 (s), 65.4 (s), 62.4 (t), 55.9 (s), 53.5 (t), 46.1 (d), 34.3 (s), 31.8 (t), 29.3 (t), 27.9 (q), 27.3 (t), 24.9 (q), 22.8 (t).

### Physicochemical data of compound AA

¹H NMRδ(ppm, CDCl₃, 500 MHz) 5.57(1H, d, *J* = 2.5 Hz), 5.27 (1H, dd, *J* = 15.3, 7.8 Hz), 5.16 (1H, dd, *J* = 15.3, 8.4 Hz), 3.94 (1H, m), 2.87 (1H, dd, *J* = 12.9,9.0 Hz), 2.70 (1H, m), 2.68 (1H, m), 2.52 (1H, m), 2.50 (1H, m), 2.33 (1H, m), 2.23 (1H, m), 2.09 (1H, m), 1.93 (2H, m), 1.90 (1H, m), 1.87 (1H, m), 1.71 (1H, ddd, *J* = 13.1, 13.1, 5.3 Hz), 1.62 (1H, m), 1.54 (1H, m), 1.51 (1H, m), 1.49 (1H, m), 1.48 (1H, m), 1.44 (3H, s), 1.43 (1H, m), 1.04 (3H, d, *J* = 6.6 Hz), 0.92 (3H, d, *J* = 6.8 Hz), 0.88 (3H, s), 0.84 (3H, d, *J* = 6.8 Hz), 0.83 (3H, d, J =6.8 Hz).
¹³C NMRδ(ppm, CDCl₃, 125 MHz) 204.6 (s), 203.6 (s), 165.3 (s), 156.0 (s), 134.5 (d), 133.0 (d), 117.4 (d), 82.7 (s), 70.2 (d), 57.5 (d), 55.4 (d), 47.8 (t), 46.2 (s), 42.9 (d), 40.1 (d), 38.8 (t), 37.7 (t), 33.3 (t), 33.1 (d), 29.6 (t), 29.0 (t), 22.0 (t), 21.0 (q), 20.2 (q), 20.0 (q), 19.7 (q), 17.6 (q), 12.1 (q).

### Physicochemical data of compound BB

¹H NMRδ(ppm, CDCl₃, 500 MHz) 5.52(1H, d, *J* = 2.5 Hz), 5.27 (1H, dd, *J* = 15.3, 7.8 Hz), 5.16 (1H, dd, *J* = 15.3, 8.6 Hz), 4.47 (1H, m), 3.12 (1H, dd, *J* = 13.6, 3.7 Hz), 2.69 (1H, m), 2.52 (2H, m), 2.40 (1H, ddd, *J* = 13.6, 4.8, 1.6 Hz), 2.24 (1H, m), 2.18 (1H, m), 2.10 (1H, m), 2.06 (1H, m), 2.02 (1H, m), 1.90 (1H, m), 1.87 (1H, m), 1.72 (1H, m), 1.68 (1H, m), 1.61 (1H, m), 1.51 (1H, m), 1.49 (1H, m), 1.48 (1H, m), 1.46 (3H, s), 1.45 (1H, m), 1.04 (3H, d, J = 6.6 Hz), 0.92 (3H, d, *J* = 6.9 Hz), 0.89 (3H, s), 0.84 (3H, d, *J* = 6.8 Hz), 0.83 (3H, d, *J* = 6.8 Hz).
¹³C NMRδ(ppm, CDCl₃, 125 MHz) 205.4 (s), 203.7 (s), 165.4 (s), 154.6 (s), 134.5 (d), 133.0 (d), 117.5 (d), 84.4 (s), 70.4 (d), 57.4 (d), 55.5 (d), 46.5 (t), 46.1 (s), 42.9 (d), 40.1 (d), 38.7 (t), 37.7 (t), 34.6 (t), 33.1 (d), 29.0 (t), 28.1 (t), 22.0 (t), 21.1 (q), 20.4 (q), 20.0 (q), 19.7 (q), 17.6 (q), 12.2 (q).

### EXAMPLE 5

### Proliferation promoting agent for neural stem cells

By preparing a DMSO solution of the compound A (0.1 mmol/L) in the usual way, a proliferation promoting agent for neural stem cells containing the compound A was obtained.

### EXAMPLE 6

### Proliferation promoting agent for neural stem cells

By preparing a DMSO solution of the compound C (0.5 mmol/L) in the usual way, a proliferation promoting agent for neural stem cells containing the compound C was obtained.

### EXAMPLE 7

### Proliferation promoting agent for neural stem cells

By preparing a DMSO solution of the compound E (0.1 mmol/L) in the usual way, a proliferation promoting agent for neural stem cells containing the compound E was obtained.

### [Reference Example 2]

### Production of compounds A1 to A15 and A

The compounds A1 to A15 were produced by the method described in Japanese Published Examined Patent Application No. 5909/96.

### [Reference Example 3]

### Production of compound A

The compound A was obtained by the method described in Example 1, by culturing a filamentous fungus belonging to the genus *Penicillium* and being capable of producing the compound (*Penicillium paxili* KAC-1843 (FERM p-8581)) and isolating and purifying it from the culture.

### INDUSTRIAL APPLICABILITY

The present invention can provide a compound produced by *Penicillium* sp. CND1007 which is useful as a proliferation promoting agent for neural stem cells and neural progenitor cells, or a related compound thereof, or a pharmaceutically acceptable salt thereof, a proliferation promoting agent for neural stem cells containing the same, and the like.

## Claims

1. A proliferation promoting agent for neural stem cells, which comprises a compound produced by *Penicillium* sp. CND1007 (FERM BP-10917) or a pharmaceutically acceptable salt thereof, as an active ingredient.

2. The proliferation promoting agent for neural stem cells according to claim 1, wherein the compound produced by *Penicillium* sp. CND1007 is a compound represented by any one of the following formulae (A) to (BB).

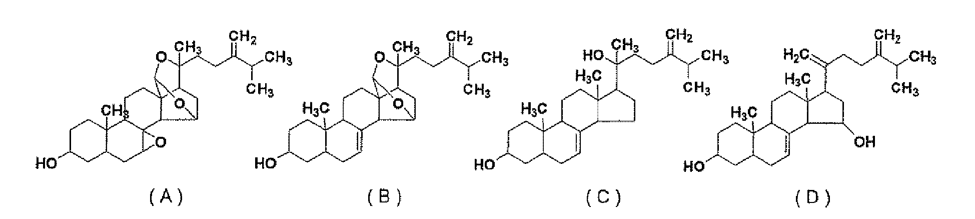

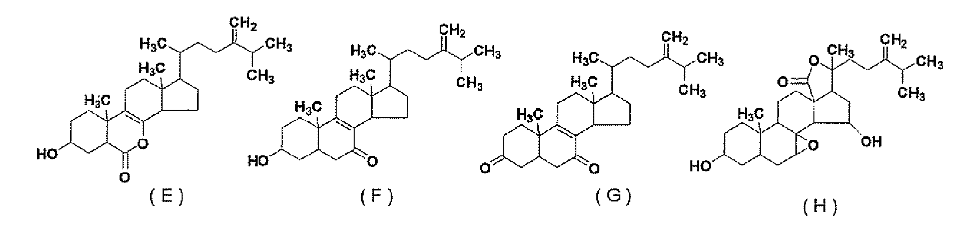

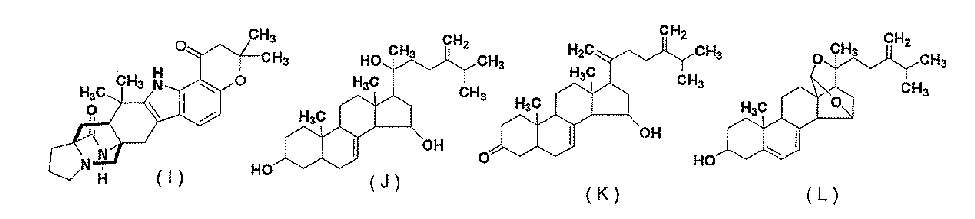

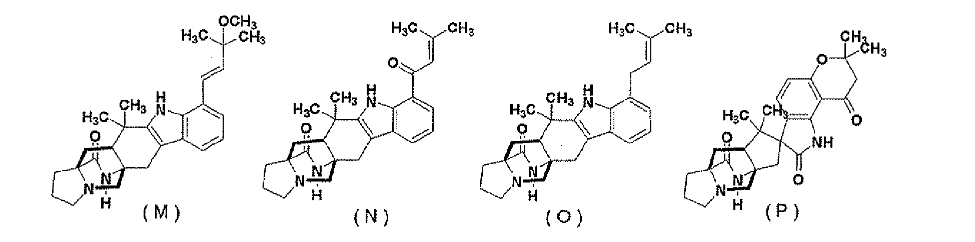

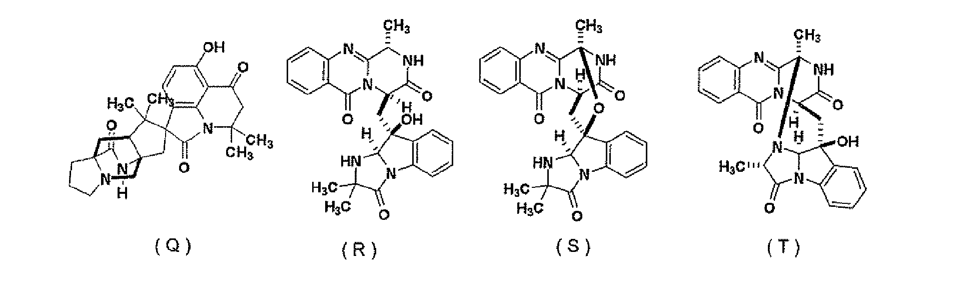

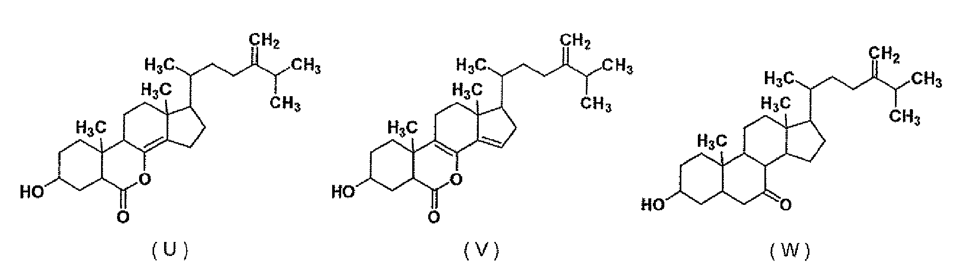

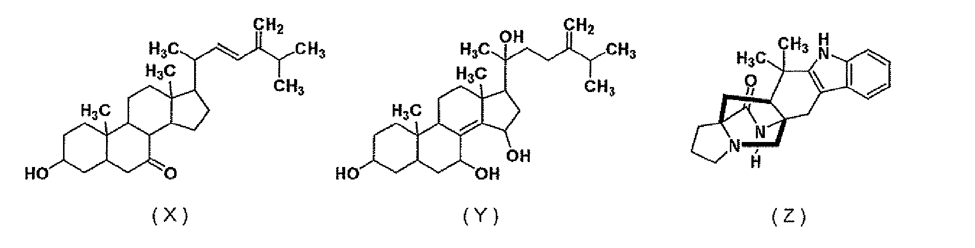

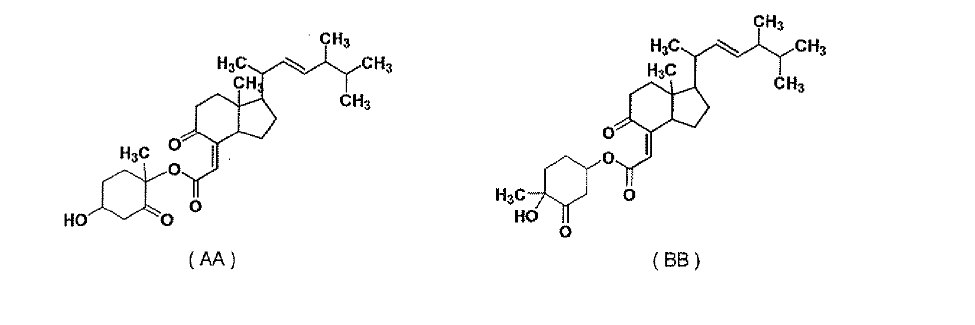

3. A method for producing neural stem cells, which comprises culturing a neural stem cells to proliferate the neural stem cells in the presence of the compound described claim 1 or 2 or a pharmaceutically acceptable salt thereof, and harvesting the neural stem cells from the culture.

4. A compound represented by any one of the following formulae (B) to (BB) or a pharmaceutically acceptable salt thereof.

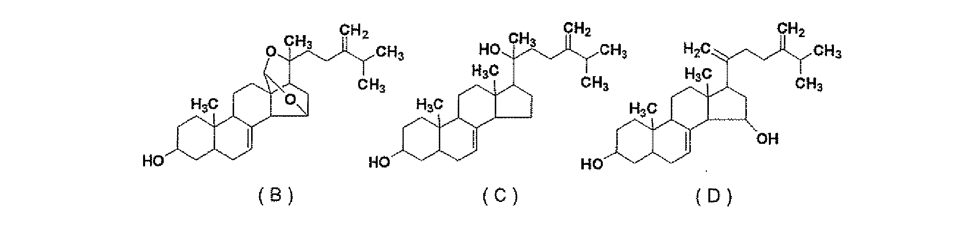

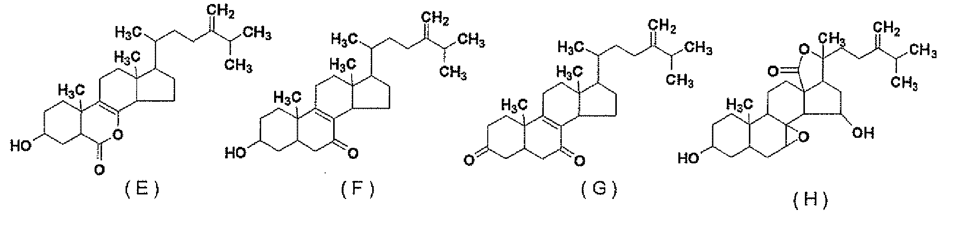

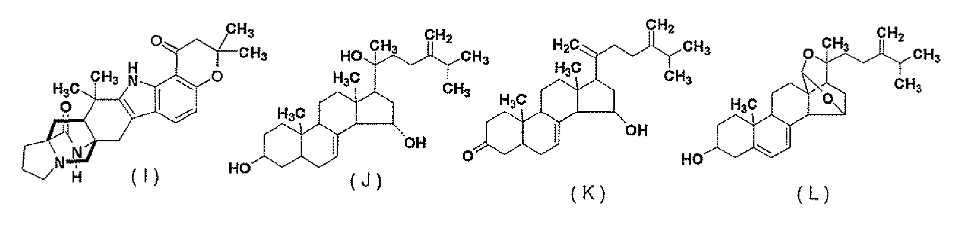

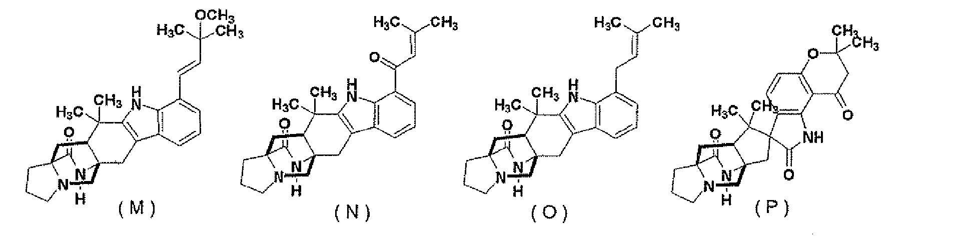

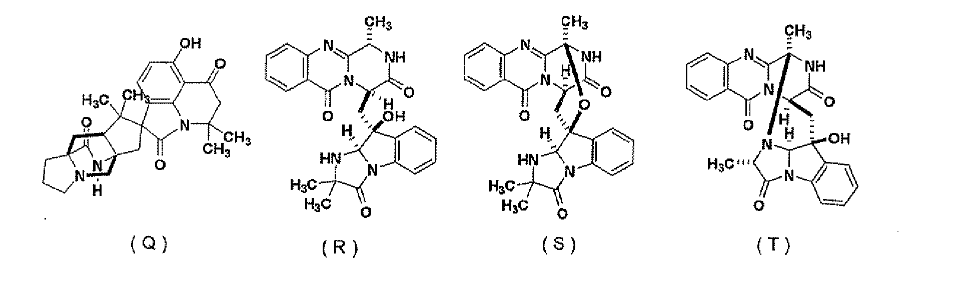

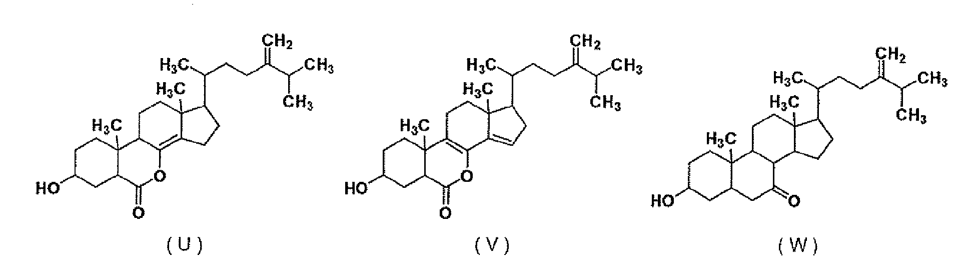

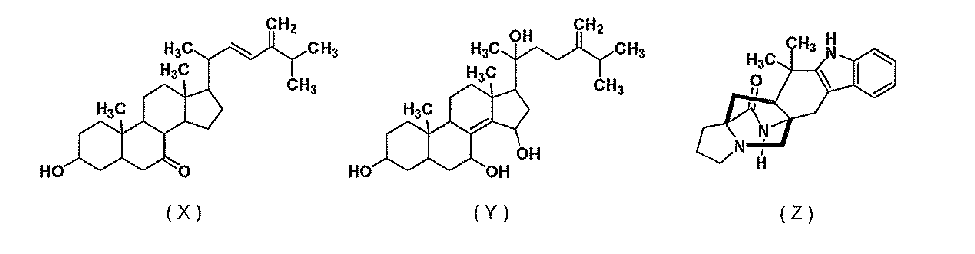

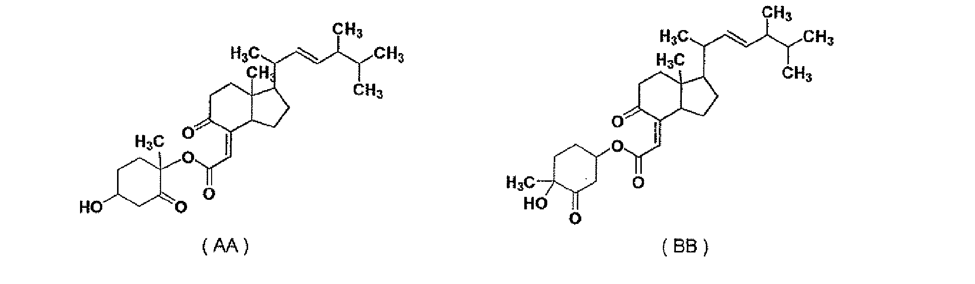

5. A method for promoting proliferation of neural stem cells, which comprises allowing the neural stem cells to contact with a compound produced by *Penicillium* sp. CND1007 or a pharmaceutically acceptable salt thereof.

6. The method according to claim 5, wherein the compound produced by *Penicillium* sp. CND1007 is a compound represented by any one of the formulae (A) to (BB) described in claim 2.

7. Use of a compound produced by *Penicillium* sp. CND 1007 or a pharmaceutically acceptable salt thereof for the manufacture of a proliferation promoting agent for neural stem cells.

8. The use according to claim 7, wherein the compound produced by *Penicillium* sp. CND1007 is a compound represented by any one of the formulae (A) to (BB) described in claim 2.
